# EUROPEAN PATENT APPLICATION

(11) **EP 4 685 136 A1**
(43) Date of publication of application: **28.01.2026**
(21) Application number: 23930062.7
(22) Date of filing: 08.12.2023
(51) Int. Cl.: C07C 229/16, C07C 323/12, C07C 237/12, C07C 233/36, A61K 9/127, A61K 47/18, A61K 47/20

(54) **AMINO LIPID, AND LIPID NANOPARTICLES AND USE THEREOF**

(30) Priority: 31.03.2023 CN 202310338416
(71) Applicant: Shenzhen MagicRNA Biotechnology Co., Ltd., Shenzhen, Guangdong 518107 (CN)
(72) Inventor: FANG, Wanyin, Shenzhen, Guangdong 518107 (CN); QIN, Haoyong, Shenzhen, Guangdong 518107 (CN); XU, Yan, Shenzhen, Guangdong 518107 (CN); WU, Qixin, Shenzhen, Guangdong 518107 (CN)
(74) Representative: Lorenz Seidler Gossel Part. mbB
(86) International application number: PCT/CN2023/137365
(87) International publication number: WO 2024/198497

(57) **Abstract**

The present invention provides an amino lipid, and lipid nanoparticles (LNPs) and a use thereof, the amino lipid having a structure represented by general formula (I), or an isomer, pharmaceutically acceptable salt, prodrug or solvate of the amino lipid. The present invention further provides LNPs containing the amino lipid. According to the present invention, the amino lipid having a structure represented by general formula (I) is used as an ionizable lipid compound, and the LNPs are obtained by means of self-assembly of the ionizable lipid compound, a steroid, a neutral lipid, and a polymer-bonded lipid. The LNPs can further improve the translation expression level of a nucleic acid load in cells, improve the effect of a nucleic acid-LNP preparation, and enable the nucleic acid-LNP preparation to provide a theoretical basis for personalized treatment.

## Description

### Technical Field

The present invention relates to the technical field of biochemistry, and in particular to an amino lipid, a lipid nanoparticle and use thereof.

### Background Art

Lipid nanoparticles (LNPs) are a novel biomolecular delivery technology for nucleic acids, and LNPs typically consist of four components: (1) ionizable lipids, which can self-assemble with mRNA to form viral-sized particles and release the mRNA from the endosome to the cytoplasm; (2) polymer-conjugated lipids, which can increase the half-life of the LNPs in the bloodstream; (3) steroids, which can increase the stability of nanoparticles; and (4) neutral phospholipids, which contribute to the formation of lipid bilayer structures.

LNP protects both mRNA from being broken down by RNA enzymes and mRNA molecules from being recognized by TLRs, preventing over-activation of the innate immune system. Among the components of LNPs, the selection of ionizable lipids has the greatest impact on LNP. Ionizable lipids play a role in promoting cellular uptake as well as helping drug molecules to escape from endosomes, and at the same time affect the encapsulation efficiency of nucleic acid drugs, the efficiency of nucleic acid drug delivery in vivo as well as cytotoxicity and the like.

Currently, there have been vaccines against COVID-19 neocoronavirus from Moderna and BioNtech & Pfizer, both of which use LNP technology to deliver mRNA drugs, thus realizing the prevention of COVID-19 neocoronavirus, demonstrating the great potential of the application of LNP in the field of mRNA drugs and vaccines. However, at present, LNP still has a low encapsulation efficiency, low endosome escape rate, low expression level, poor safety and other shortcomings, and the development direction of LNP delivery system is mainly focused on ionizable lipids and formulations.

Accordingly, the development of novel ionizable lipid compounds is of great significance to the development of nucleic acid drugs.

### Summary of the Invention

In the first aspect, the present invention provides an amino lipid having the structure of formula (I), or an isomer, a pharmaceutically acceptable salt, a prodrug or a solvate thereof: 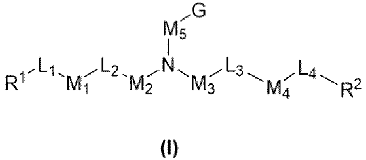
wherein G is selected from H, OR, CN, -C(=O)OR', -OC(=O)R', -C(=O)NR'R", -NR'C(=O)R", -NR'R", or a cycloalkyl comprising at least one heteroatom, wherein the substitutable carbon atoms or heteroatoms in the cycloalkyl are unsubstituted or substituted by one or more groups of hydroxyl, C₁-C₄ alkyl, C₂-C₄ alkenyl, C₃-C₈ cycloalkyl, or C₃-C₈ cycloalkenyl;
M₁, M₂, M₃, and M₄ are identical or different from each other, and each independently selected from C₁-C₂₄ alkylene, C₃-C₂₄ cycloalkylene, C₂-C₂₄ alkenylene, or C₃-C₂₄ cycloalkenylene;
R¹ and R² are identical or different from each other, and each independently selected from H, C₁-C₂₄ alkyl, C₃-C₂₄ cycloalkyl, C₂-C₂₄ alkenyl, or C₃-C₂₄ cycloalkenyl;
L₁, L₂, L₃, and L₄ are identical or different from each other, and each independently selected from -C(=O)O-, -OC(=O)-, -C(=O)S-, -SC(=O)-, -C(=O)NR-, -NRC(=O)-, -S(=O)-, -OS(=O)₂-, -S(=O)₂O-, -O-, -S-, or -S-S-;
R, R', and R" are identical or different from each other, and each independently selected from H, C₁-C₁₀ alkyl, C₃-C₁₀ cycloalkyl, C₃-C₁₀ alkenyl, C₃-C₁₀ cycloalkenyl, C₁-C₁₀ alkyl terminated with a tertiary amino group, C₃-C₁₀ cycloalkyl terminated with a tertiary amino group, C₃-C₁₀ alkenyl terminated with a tertiary amino group, or cycloalkyl comprising at least one heteroatom, the cycloalkyl being unsubstituted or substituted with one or more groups of C₁-C₄ alkyl, C₂-C₄ alkenyl, C₃-C₈ cycloalkyl, or C₃-C₈ cycloalkenyl;
M₅ groups are each independently selected from a single bond, C₁-C₁₆ alkylene, C₂-C₁₆ alkenylene, C₃-C₈ cycloalkylene, or C₃-C₈ cycloalkenylene.

In the second aspect, the present invention provides a method for preparing the above-mentioned amino lipid. The synthesis mainly involves three reaction steps: the first step is a ring-opening reaction; the second step is a condensation reaction; and the third step is a substitution reaction.

In some embodiments of the present invention, the method for preparing the amino lipid comprises the following steps:
S1: subjecting an epoxide compound and a carboxylic acid to ring-opening reaction to prepare R¹-L₁-M₁-OH as intermediate 1;
S2: subjecting the intermediate 1 and a carboxylic acid compound as a starting material to a condensation reaction in the presence of a condensation agent to give R¹-L₁-M₁-L₂-M₂-leaving group as intermediate 2; and
S3: subjecting the intermediate 2 and an amino compound as a starting material to one or more substitution reactions, to obtain the target product; or
it comprises the following steps:
   S1': performing oxidation reaction of a diol undergoing TBS protection, and subjecting the oxidation product and a Grignard reagent to addition reaction, to give HO-M₁-OTBS as intermediate 1';
   S2': subjecting the intermediate 1' and a carboxylic acid compound to a condensation reaction in the presence of a condensation agent, to give TBSO-M₁-L₂-M₂-leaving group as intermediate 2';
   S3': removing the protective group of TBS from the intermediate 2' TBSO-M₁-L₂-M₂-leaving group, allowing the resultant to undergo a condensation reaction with a carboxylic acid in the presence of a condensation agent, to generate R¹-L₁-M₁-L₂-M₂-leaving group as intermediate 2; and
   S4': subjecting the intermediate 2 and an amino compound as a starting material to one or more substitution reactions, to obtain the target product.

In the third aspect, the present invention provides a lipid nanoparticle comprising any of the above-mentioned amino lipid.

The lipid nanoparticle according to the present invention further comprises a steroid, a neutral lipid and/or a polymer-conjugated lipid;

The polymer-conjugated lipid has a chemical formula of P-Y-L, in which P is a hydrophilic polymer moiety, Y is an optional linker, and L is a lipid moiety.

In the fourth aspect, the present invention provides a pharmaceutical composition comprising the aforementioned lipid nanoparticle and a pharmaceutically acceptable carrier.

In the fifth aspect, the present invention further provides a method for treating or preventing infectious diseases, cancer, genetic diseases, allergy, toxicity, and autoimmune diseases using the aforementioned amino lipid or lipid nanoparticle or pharmaceutical composition.

The present invention further provides a method for gene therapy, genetic vaccination, antisense therapy, delivery of nucleic acids, or treatment through RNA interference, by using the aforementioned amino lipid, lipid nanoparticle or pharmaceutical composition.

The present invention further provides use of the aforementioned amino lipid or lipid nanoparticle in the preparation of a medicament for treating or preventing infectious diseases, cancer, genetic diseases, allergy, toxicity, and autoimmune diseases.

The present invention further provides use of the aforementioned amino lipid or lipid nanoparticle in the preparation of a medicament for gene therapy, genetic vaccination, antisense therapy, delivery of nucleic acids, or treatment through RNA interference.

In the sixth aspect, the present invention provides a method for delivering a medicament to a subject, comprising administering to the subject a medicament formulated in the aforementioned lipid nanoparticle.

The amino lipid of the present invention is self-assembled with steroids, neutral lipids, and polymer-conjugated lipids to form LNPs, which can further enhance the translational expression level of the loaded nucleic acid within cells, improve the efficacy of nucleic acid-LNP formulations, and provide a theoretical foundation for personalized treatment with nucleic acid-LNP formulations.

### Brief Description of the Drawings

Figure 1 shows the ¹H-NMR spectrum of E12LA6B603 in Example 2;
Figure 2 shows the tumour size monitoring curve of the tumour-bearing mice after intramuscular injection of the OVA mRNA vaccine in Example 39;
Figure 3 shows the survival curve of the tumour-bearing mice after intramuscular injection of the OVA mRNA vaccine in Example 39.

### Best Mode of the Invention

### Definitions

For clarity and ease of reading, the following scientific background information and definitions are provided. Any technical features mentioned or disclosed herein may be part of each embodiment of the present invention or may be read into each embodiment of the present invention. Other definitions and explanations may be provided in the context of the present invention.

Unless otherwise defined, or unless the specific context requires otherwise, all technical terms used herein have the same meaning as those commonly understood by those skilled in the relevant technical field.

Unless the context otherwise indicates or requires, the terms "include," "contain," and "comprise," as well as similar expressions, should be interpreted in this specification and claims as "including/comprising but not limited to' in an open and inclusive manner.

Expressions such as "an embodiment', "embodiments" or "a specific embodiment' mean that a particular feature, characteristic, or property, or a specific group or combination of such features, characteristics, or properties as described in conjunction with the corresponding expression, exist in at least one embodiment of the present invention. These expressions appearing in different places throughout the description do not necessarily refer to the same embodiment. Furthermore, specific features, characteristics, or properties may be combined in any suitable manner in one or more embodiments.

Unless otherwise explicitly stated in the context, the singular forms "an", "a", and "the" should be understood to include plural meanings.

When the term "neutral" is applied to compounds such as lipids or steroids, or groups or moieties, it indicates that the compound is neither cationic nor anionic, such as compounds that do not possess ionizable functional groups under physiological conditions, e.g., hydrocarbons; or it indicates that the compound is both cationic and anionic under typical physiological conditions, i.e., zwitterionic, such as typical natural phosphatidylcholine.

As used herein, "lipid" refers to a group of organic compounds that are derivatives of fatty acids (such as esters), typically characterized by being insoluble in water but soluble in many organic solvents. Lipids are typically classified into at least three types: (1) "simple lipids", including fats, oils, and waxes; (2) "complex lipids", including phospholipids and glycolipids; and (3) "derived lipids", such as steroids. Regarding glycolipids, in certain embodiments, the LNP comprises glycolipids (e.g., monosialoganglioside GM1).

In the context, the prefix "poly" refers to multiple atoms or groups in a compound that possess their respective properties. However, the absence of the prefix should not be interpreted as excluding the plural form. For example, polycationic compounds are also cationic compounds and may be referred to as cationic compounds.

The term "nucleic acid" refers to any compound containing DNA or RNA or composed of them. This term can be used for oligonucleotides.

Immune system: The immune system can protect organisms from infection. If pathogens break through an organism's physical barriers and enter it, the innate immune system provides an immediate but non-specific response. If the pathogen evades this innate response, vertebrates have a second layer of protection, the adaptive immune system. Here, the immune system adjusts its response during infection to improve its ability to recognize the pathogen. This improved response is then retained in the form of immune memory after the pathogen is eliminated, allowing the adaptive immune system to mount a faster and stronger attack each time it encounters the pathogen. Thus, the immune system comprises the innate immune system and the adaptive immune system. Each of these components includes so-called humoral and cellular components.

Adaptive Immune System: the adaptive immune system consists of highly specialized systemic cells and processes that can eliminate or prevent pathogenic growth. The adaptive immune response provides the immune system of vertebrates with the ability to recognize and remember specific pathogens (immunity) and mount a stronger attack each time they encounter the pathogen. This system is highly adaptive due to somatic hypermutation (an increase in the frequency of somatic mutations) and V(D)J recombination (irreversible genetic recombination of antigen receptor gene fragments). This mechanism allows a small number of genes to produce a large number of different antigen receptors, which are then uniquely expressed on each individual lymphocyte. Since gene rearrangement causes irreversible changes in the DNA of each cell, all descendants (offspring) of that cell inherit genes encoding the same receptor specificity, including memory B cells and memory T cells, which are key to long-lived specific immunity. The immune network theory is a theory about how the adaptive immune system works, based on the interactions between the variable regions of T cells, B cells, and the receptors of molecules produced by T cells and B cells that have variable regions.

The term "vaccine" is generally understood to refer to a preventive or therapeutic material that provides at least one antigen or antigenic function. Antigens or antigenic functions can stimulate the body's adaptive immune system to provide an adaptive immune response.

The term "antigen" generally refers to a substance that can be recognized by the immune system, preferably by the adaptive immune system, and that can trigger an antigen-specific immune response, such as by forming antibodies and/or antigen-specific T cells as part of the adaptive immune response.

The term "artificial mRNA" (sequence) can generally be understood as an mRNA molecule that does not exist naturally. In other words, artificial mRNA molecules can be understood as non-natural mRNA molecules. Such mRNA molecules may be non-natural due to their individual sequences (not naturally occurring) and/or due to other modifications, such as structural modifications of non-naturally occurring nucleotides. Typically, artificial mRNA molecules can be designed and/or produced using genetic engineering methods to correspond to the desired artificial nucleotide sequence (heterologous sequence). In this context, an artificial sequence is typically a non-naturally occurring sequence, i.e., it differs from the wild-type sequence by at least one nucleotide. The term "wild-type" can be understood as a sequence that exists in nature.

The term "pharmaceutically acceptable salt" refers to a form of a compound that does not cause significant irritation to the organism receiving the administration and does not cause the compound to lose its biological activity and properties.

The compounds described in this invention may exist in multiple crystalline forms, i.e., different crystal lattice arrangements of the same elements of the compound. Polymorphs typically have different X-ray diffraction spectra, infrared spectra, melting points, densities, hardnesses, crystal forms, optical and electrical properties, stabilities, and solubilities. Different factors such as recrystallization solvents, crystallization rates, and storage temperatures may result in recrystallization products dominated by a single crystalline form. It should be understood that the amino lipids described in the present invention include all such crystalline forms.

The compounds described in the present invention may contain chiral centers and/or axial chirality and may therefore exist in the form of racemic compounds, racemic mixtures, single enantiomers, diastereomeric compounds, and single diastereomers, as well as in the form of cis-trans isomers. Each chiral center or axial chirality will independently produce two optical isomers, and all possible optical isomers and diastereomeric mixtures, as well as pure or partially pure compounds, are included within the scope of the present invention. The term "each isomer" as defined in the present invention includes all such isomeric forms of the compounds.

The compounds described in the present invention may also exist in various hydrates or solvates, where the solvates contain stoichiometric or non-stoichiometric amounts of solvent and are selectively formed during crystallization with pharmaceutically acceptable solvents such as water or ethanol. When the solvent is water, hydrates are formed, and when the solvent is ethanol or other solvents, solvates are formed.

The term "prodrug" is also referred to as precursor drug, drug precursor, or pro-kinetic drug, etc., and refers to a compound obtained through chemical structural modification of a drug that is inactive or has low activity in vitro but releases an active drug through enzymatic or non-enzymatic conversion in vivo to exert its pharmacological effect. Prodrugs are divided into two major categories: carrier prodrugs and biological prodrugs. Carrier prodrugs are compounds with active components covalently bound to a carrier that transports them. Within the body, the carrier is removed through simple hydrolysis, allowing the active compound to exert its pharmacological effects. Carrier prodrugs are often less active or inactive compared to their parent compounds. The structure of the carrier is typically lipophilic, required to be non-toxic to the body and capable of promptly releasing the active compound. Biological prodrugs differ from the carrier prodrugs in that the active substance does not need to be temporarily bound to a carrier but instead exerts its effects through changes in its own molecular structure. Biological prodrugs themselves are inactive, and the active compounds are their metabolites within the body.

Through research, it is unexpectedly discovered that the use of the novel amino lipids and/or lipid nanoparticles provided by the present invention can effectively overcome the shortcomings of existing technologies, such as low encapsulation efficiency, low endosome escape rate, low expression levels, and poor safety, thereby promoting the development of LNPs in the fields of mRNA drugs and vaccines.

### Amino lipid

Amino lipids are preferably cationizable, i.e., when the pH is lowered below the pKa of the ionizable group of the lipid, the amino lipid becomes protonated, and when it carries a positive charge, the lipid can bond to negatively charged nucleic acids.

In one aspect, the present invention provides an amino lipid having the structure of formula (I), or an isomer, a pharmaceutically acceptable salt, a prodrug or a solvate thereof: 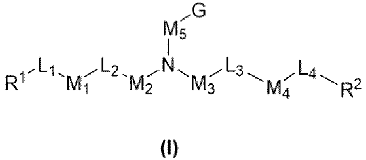
wherein G is selected from H, OR, CN, -C(=O)OR', -OC(=O)R', -C(=O)NR'R", -NR'C(=O)R", -NR'R", or a cycloalkyl (preferably 3- to 10-membered cycloalkyl, more preferably 4- to 6-membered cycloalkyl) comprising at least one heteroatom (preferably, the heteroatom is N or O, preferably 1 or 2 heteroatoms selected from N or O); the substitutable carbon atoms or heteroatoms in the cycloalkyl are unsubstituted or substituted by one or more groups of C₁-C₄ alkyl, C₂-C₄ alkenyl, C₃-C₈ cycloalkyl, or C₃-C₈ cycloalkenyl;
M₁, M₂, M₃, and M₄ are identical or different from each other, and each independently selected from C₁-C₂₄ alkylene, C₃-C₂₄ cycloalkylene, C₂-C₂₄ alkenylene, or C₃-C₂₄ cycloalkenylene;
R¹ and R² are identical or different from each other, and each independently selected from H, C₁-C₂₄ alkyl, C₃-C₂₄ cycloalkyl, C₂-C₂₄ alkenyl, or C₃-C₂₄ cycloalkenyl;
L₁, L₂, L₃, and L₄ are identical or different from each other, and each independently selected from -C(=O)O-, -OC(=O)-, -C(=O)S-, -SC(=O)-, -C(=O)NR-, -NRC(=O)-, -S(=O)-, -OS(=O)₂-, -S(=O)₂O-, -O-, -S-, or -S-S-;
R, R', and R" are identical or different from each other, and each independently selected from H, C₁-C₁₀ alkyl (preferably C₁-C₆ alkyl, more preferably C₁-C₄ alkyl), C₃-C₁₀ cycloalkyl, C₃-C₁₀ alkenyl, C₃-C₁₀ cycloalkenyl, C₁-C₁₀ alkyl terminated with a tertiary amino group, C₃-C₁₀ cycloalkyl terminated with a tertiary amino group, C₃-C₁₀ alkenyl terminated with a tertiary amino group, or cycloalkyl comprising at least one heteroatom, the cycloalkyl being unsubstituted or substituted with one or more groups of C₁-C₄ alkyl, C₂-C₄ alkenyl, C₃-C₈ cycloalkyl, or C₃-C₈ cycloalkenyl; preferably, C₁-C₁₀ alkyl terminated with -N(C₁₋₆ alkyl)₂, or C₁-C₁₀ alkyl terminated with a 3- to 8-membered cycloalkyl (preferably a 4- to 6-membered cycloalkyl) containing 1 to 2 ring nitrogen atoms, wherein the C₃-C₁₀ cycloalkyl and 3- to 8-membered cycloalkyl (preferably 4- to 6-membered cycloalkyl) may optionally be substituted with C₁-C₆ alkyl.
M₅ is selected from a single bond, C₁-C₁₆ alkylene, C₂-C₁₆ alkenylene, C₃-C₈ cycloalkylene, or C₃-C₈ cycloalkenylene.

It should be noted that, unless explicitly prohibited, the alkyl, alkenyl, alkylene, and alkenylene groups mentioned in this invention, which contain multiple carbon atoms, may be either straight- or branched-chain. Additionally, the alkyl, alkenyl, alkylene, alkenylene, cycloalkyl, cycloalkenyl, cycloalkylene, and cycloalkenylene mentioned in this invention may be unsubstituted or substituted, with the substituents being any suitable substituents, i.e., any straight- or branched-chain alkyl, aryl, heteroalkyl, or heteroaromatic structures, which may optionally contain other functional groups, such as ester or amide groups.

In some preferred embodiments, G is selected from H, OR, or NR'R", or cycloalkyl comprising at least one heteroatom, wherein the heteroatom is O or N; wherein R, R', and R" are identical or different from each other, and each independently selected from H, C₁-C₈ alkyl, C₃-C₈ cycloalkyl, C₃-C₈ alkenyl, or C₃-C₈ cycloalkenyl.

In further preferred embodiments, G is selected from H, OR, or NR'R", wherein R' and R" are identical or different from each other, and each independently selected from H or C₁-C₄ alkyl; i.e., NR'R" may be NH₂, NHCH₃, NHC₂H₅, NHC₃H₇, NHC₄H₉, N(CH₃)₂, CH₃-N-C₂H₅, CH₃-N-C₃H₇, CH₃-N-C₄H₉, C₂H₅-N-C₂H₅, C₂H₅-N-C₃H₇, C₂H₅-N-C₄H₉, C₃H₇-N-C₃H₇, C₃H₇-N-C₄H₉ or C₄H₉-N-C₄H₉.

Alternatively, G is selected from substituted or unsubstituted 5-membered oxacycloalkyl, substituted or unsubstituted 5-membered azacycloalkyl, substituted or unsubstituted 6-membered azacycloalkyl, substituted or unsubstituted 6-membered diazocycloalkyl, or substituted or unsubstituted 6-membered azaoxacycloalkyl. Preferably, the 5-membered oxacycloalkyl is 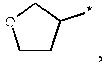 the 5-membered azacycloalkyl is 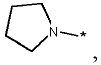 the 6-membered azacycloalkyl is 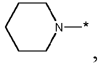 the 6-membered diazocycloalkyl is 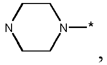 the 6-membered azaoxacycloalkyl is 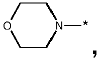 wherein the site of * is linked with Ms.

In some embodiments, when G is a substituted 6-membered diazocycloalkyl, the substituent is positioned on the nitrogen atom that is not linked with Ms.

In some preferred embodiments, G is selected from H, OR, CN, -C(=O)OR', -OC(=O)R', -C(=O)NR'R", -NR'C(=O)R", NR'R", or a cycloalkyl (preferably a 3- to 10-membered cycloalkyl, more preferably a 4- to 6-membered cycloalkyl) containing 1 or 2 ring nitrogen atoms; the substitutable carbon atoms or heteroatoms in the cycloalkyl are unsubstituted or substituted by one or more groups of C₁-C₄ alkyl, C₂-C₄ alkenyl, C₃-C₈ cycloalkyl or C₃-C₈ cycloalkenyl. R, R', and R" are identical or different from each other, and each independently selected from H, C₁-C₆ alkyl, C₃-C₁₀ cycloalkyl, C₁-C₁₀ alkyl terminated with -N(C₁₋₆ alkyl)₂, or C₁-C₁₀ alkyl terminated with a 4- to 6-membered cycloalkyl containing 1 to 2 ring nitrogen atoms, the C₃-C₁₀ cycloalkyl and 4- to 6-membered cycloalkyl may optionally be substituted with C₁-C₆ alkyl.

In some embodiments, M₅ is selected from a single bond, C₂-C₁₆ alkylene, C₂-C₁₆ alkenylene, C₄-C₈ cycloalkylene or C₃-C₈ cycloalkenylene.

More preferably, M₅ is selected from a single bond, C₂-C₁₆ alkylene, or C₄-C₆ cycloalkylene. Most preferably, M₅ is selected from a single bond, C₂-C₆ alkylene or C₄-C₆ cycloalkylene; or selected from C₃-C₅ alkylene.

In a preferred embodiment, in the formula (I), the 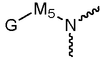 formed by linking of M₅ and G is one selected from A1 to A38:

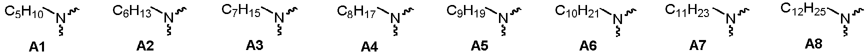

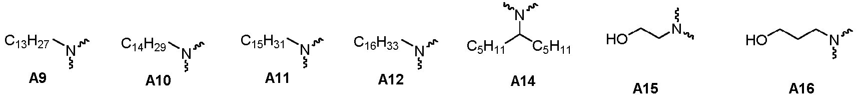

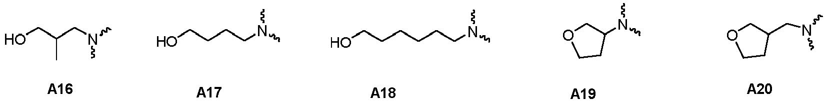

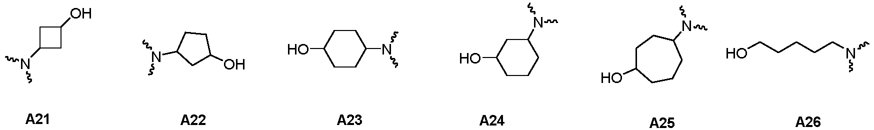

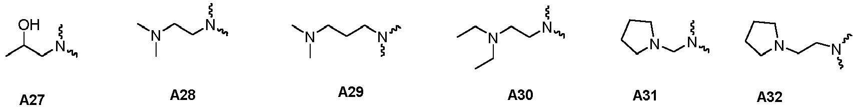

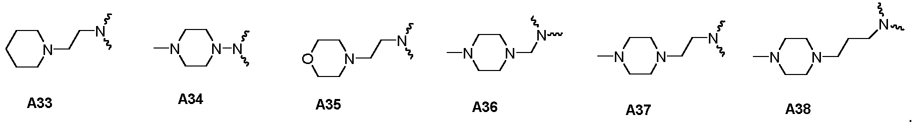

Further preferably, the 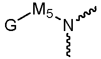 is one selected from A1-A18, A22-A24, and A28-A38.

More preferably, the 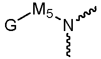 is one selected from A15, A16, A17, A23, A29, A30, A33, A37, A38, and A42.

In some embodiments of the present invention, in the formula (I), the 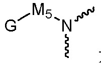 formed by linking of M₅ and G is one selected from A39 to A52:

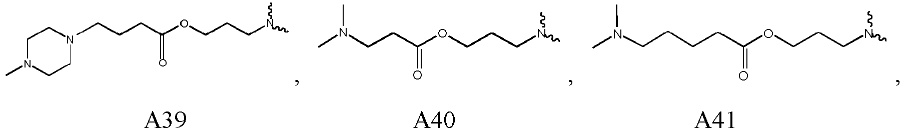

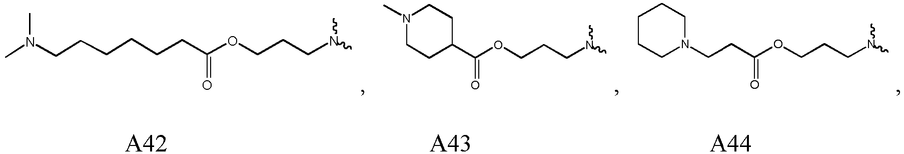

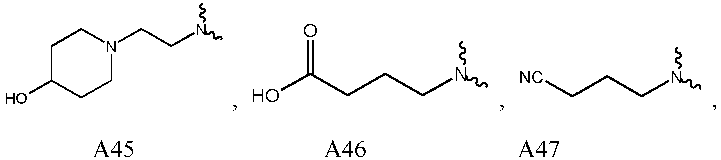

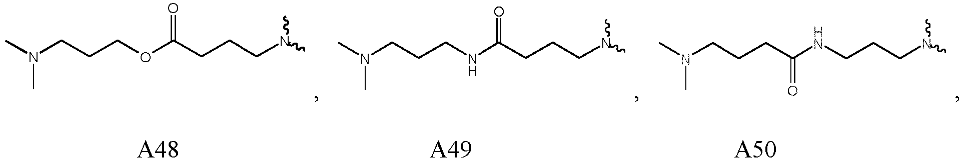

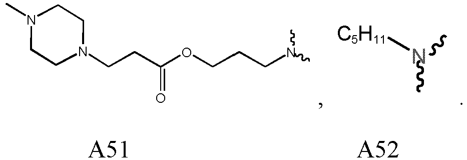

In some embodiments of the present invention, L₁, L₂, L₃, and L₄ are identical or different, and independently selected from -C(=O)O-, -OC(=O)-, -C(=O)NR-, or -NRC(=O)-,
wherein when L₁, L₂, L₃, and L₄ are independently selected from -C(=O)NR- or -NRC(=O)-, R is independently selected from H or C₁-C₁₀ alkyl. Preferably, R is H.

In a preferred embodiment of the present invention, L₁ and L₄ are the same, and are -C(=O)O- or -OC(=O)-.

In some embodiments of the present invention, M₁, M₂, M₃, and M₄ are identical or different, M₁ and M₄ are each independently selected from branched-chain C₄-C₂₂ alkylene, branched-chain C₄-C₂₂ cycloalkylene, branched-chain C₄-C₂₂ alkenylene, or branched-chain C₄-C₂₂ cycloalkenylene, and M₂ and M₃ are each independently selected from C₄-C₂₂ alkylene, C₄-C₂₂ cycloalkylene, C₄-C₂₂ alkenylene, or C₄-C₂₂ cycloalkenylene.

Further preferably, M₁, M₂, M₃, and M₄ are identical or different, and M₁ and M₄ are each independently selected from branched-chain C₄-C₂₂ alkylene, or branched-chain C₄-C₂₂ alkenylene, preferably, branched-chain C₆-C₁₆ alkylene; and M₂ and M₃ are each independently selected from C₄-C₂₂ alkylene or C₄-C₂₂ alkenylene, preferably C₃-C₈ alkylene.

More preferably, in some embodiments of the present invention, M₂ is the same as M₃ and is a C₄-C₂₂ alkylene.

In some embodiments of the present invention, M₁ is the same as M₄, and is a branched-chain C₄-C₂₂ alkylene.

In some embodiments of the present invention, R¹ and R² are identical or different, and each independently selected from C₄-C₂₂ alkyl or C₄-C₂₂ alkenyl, preferably C₅-C₁₂ alkyl.

In preferred embodiments, the R¹-L₁-M₁-L₂-M₂- fragment is R¹-C(=O)O-M₁-OC(=O)-M₂-, the R²-L₄-M₄-L₃-M₃- fragment is R²-C(=O)O-M₄-OC(=O)-M₃-. Further preferably, M₁, M₂, M₃, and M₄ are identical or different, and M₁ and M₄ are each independently selected from branched-chain C₄-C₂₂ alkylene, or branched-chain C₄-C₂₂ alkenylene; and M₂ and M₃ are each independently selected from C₄-C₂₂ alkylene or C₄-C₂₂ alkenylene; R¹ and R² are identical or different, and each independently selected from C₄-C₂₂ alkyl or C₄-C₂₂ alkenyl.

Those skilled in the art may combine the above preferred embodiments for different groups in accordance with common knowledge to obtain more preferred embodiments of the compounds of the present invention.

In a preferred embodiment of the present invention, the structure of the formula (I) is one selected from the following structures:

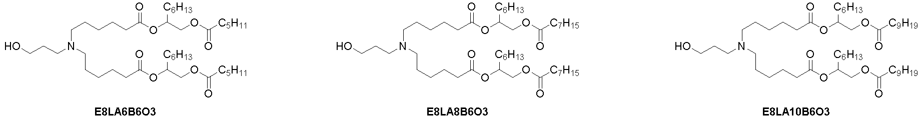

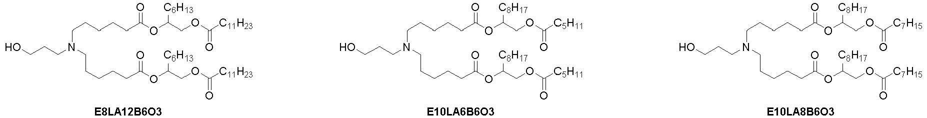

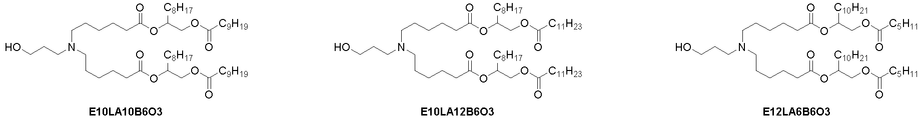

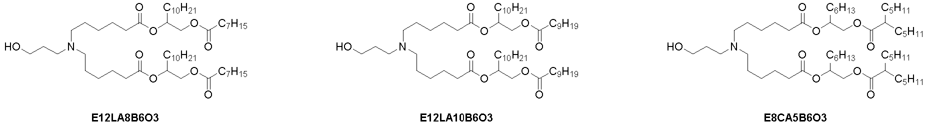

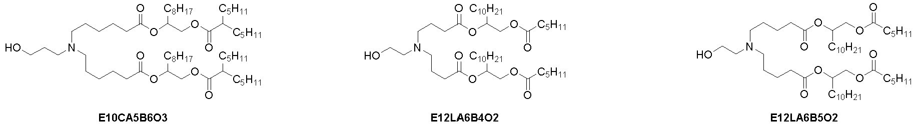

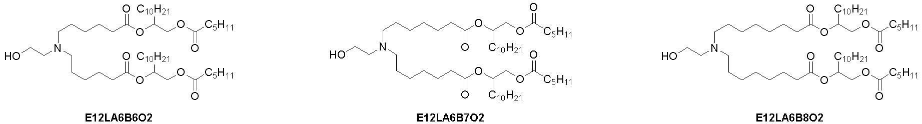

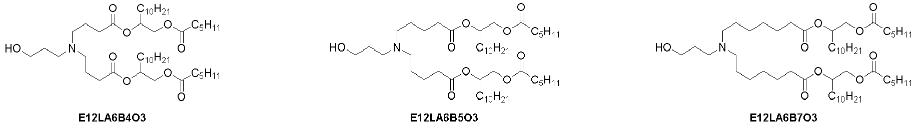

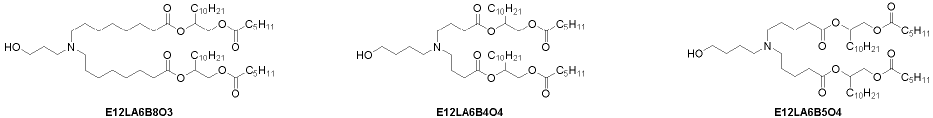

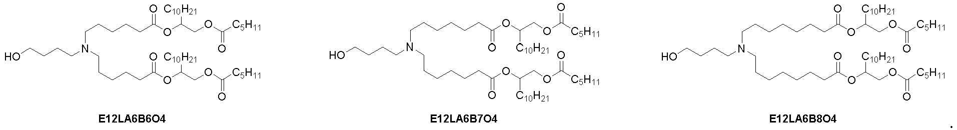

In a preferred embodiment of the present invention, the structure of the formula (I) is one selected from the following structures:

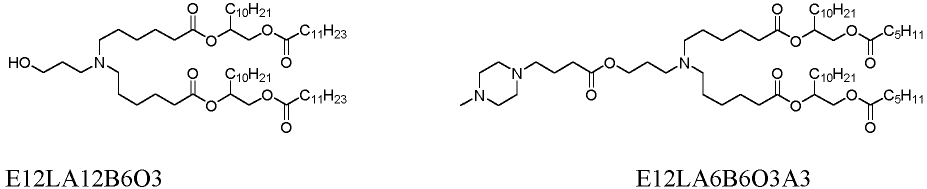

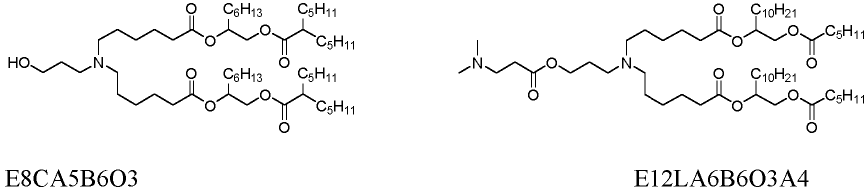

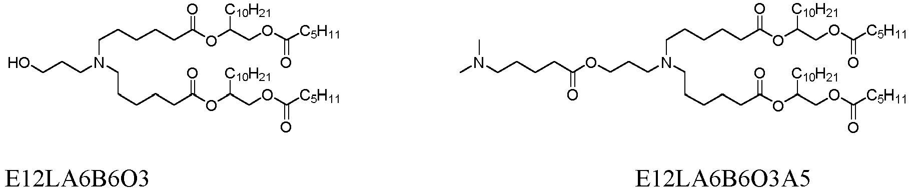

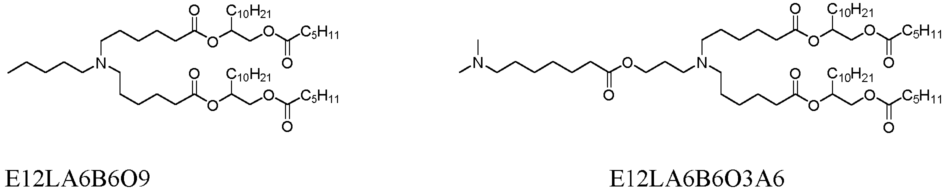

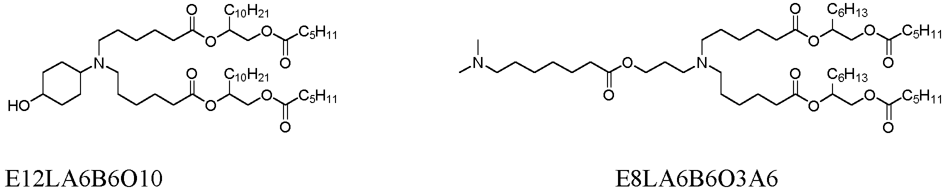

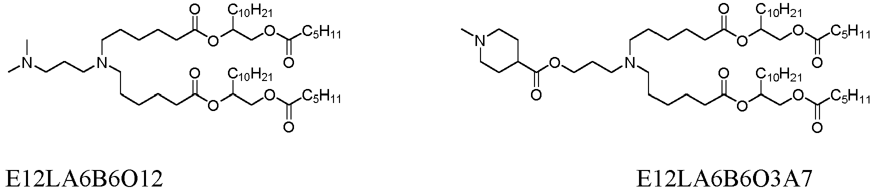

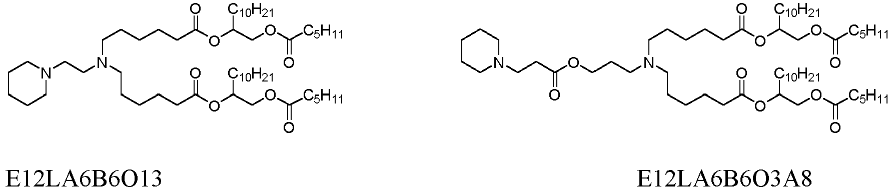

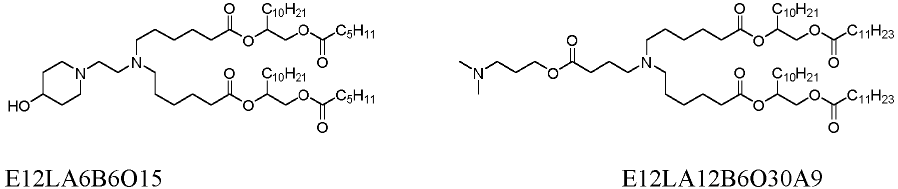

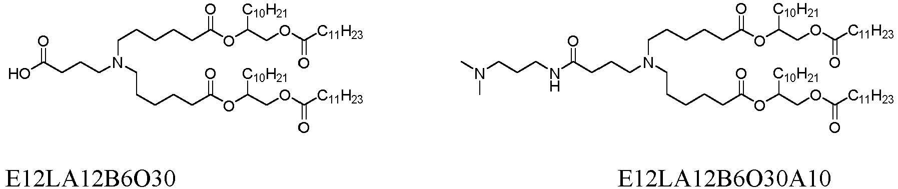

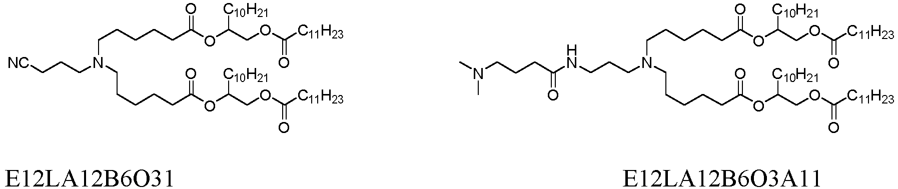

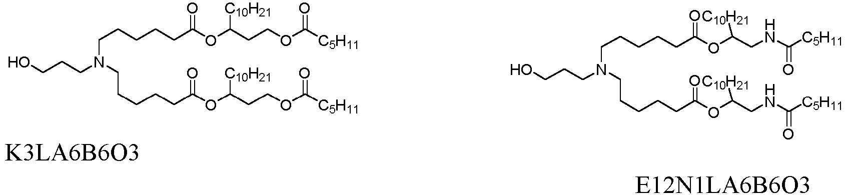

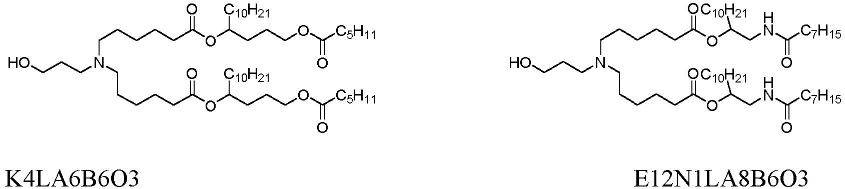

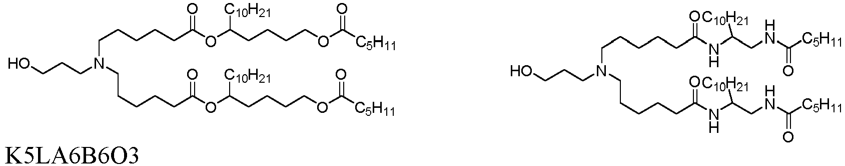

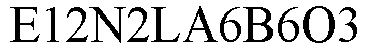

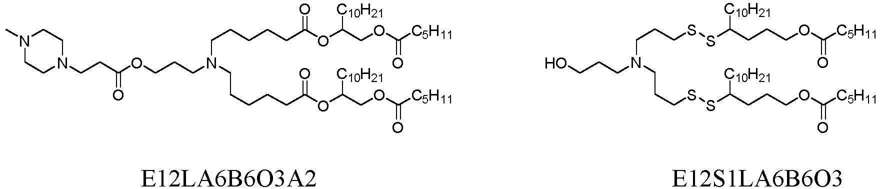

Those skilled in the art can combine the above preferred embodiments for different groups based on common knowledge to obtain more preferable embodiments of the compounds of the present invention.

### Preparation method for the amino lipid

The present invention provides a method for preparing the aforementioned amino lipid, comprising the following steps:
S1: subjecting an epoxide compound and a carboxylic acid to ring-opening reaction to prepare R¹-L₁-M₁-OH as intermediate 1;
S2: subjecting the intermediate 1 and a carboxylic acid compound as a starting material to a condensation reaction in the presence of a condensation agent to give R¹-L₁-M₁-L₂-M₂-leaving group as intermediate 2; and
S3: subjecting the intermediate 2 and an amino compound as a starting material to one or more substitution reactions, to obtain the target product; or
comprising the following steps:
   S1': performing oxidation reaction of a diol undergoing TBS protection, and subjecting the oxidation product and a Grignard reagent to addition reaction, to give HO-M₁-OTBS as intermediate 1';
   S2': subjecting the intermediate 1' and a carboxylic acid compound to a condensation reaction in the presence of a condensation agent, to give TBSO-M₁-L₂-M₂-leaving group as intermediate 2';
   S3': removing the protective group of TBS from the intermediate 2' TBSO-M₁-L₂-M₂-leaving group, allowing the resultant to undergo a condensation reaction with a carboxylic acid in the presence of a condensation agent, to generate R¹-L₁-M₁-L₂-M₂-leaving group as intermediate 2; and
   S4': subjecting the intermediate 2 and an amino compound as a starting material to one or more substitution reactions, to obtain the target product.

Preferably, the carboxylic acid in step S2' is H-L₂-M₂-leaving group.

Preferably, the carboxylic acid in step S3' is R¹-L₁-H.

Preferably, the leaving group is a halogen.

Preferably, all intermediates and target products are purified by column chromatography.

In some embodiments, the preparation method comprises the following steps:
ring-opening reaction: carboxylic acid compound (1.0 eq), epoxy compound as a starting material (0.6-3.0 eq), ferric trichloride (0.5-10 mol%), pyridine (0.2-20 mol%) are mixed, and stirred at room temperature overnight. After the reaction is finished, column chromatography purification is carried out to give intermediate 1, with a yield of 56%-99.0%.

Condensation reaction: Intermediate 1 (1.0 eq), halocarboxylic acid (0.6-3.0 eq), EDCI-HCl (1.0-6.0 eq), DMAP (0.05-0.5 eq), DIPEA (1.0-8.0 eq), and DCM are mixed, and stirred at room temperature overnight, and column chromatography purification is performed to give the intermediate 2, with a yield of 46.0%-96.4%.

Substitution reaction: Intermediate 2 (1.0-5.0 eq), potassium carbonate (1.0-5.0 eq), amine compounds (1.0 eq), sodium iodide (1.0-3.0 eq), and acetonitrile are mixed, followed by stirring overnight at 20-100°C, and column chromatography purification, to obtain amino lipid, with a yield of 32.0%-92.1%.

In some embodiments, the preparation method includes the following steps:
Addition reaction: the diol subjected to TBS protection is subjected to oxidation reaction with PCC. The oxidation product (0.9-1.2 eq) is stirred in anhydrous tetrahydrofuran at -20°C for 2-20 minutes, followed by addition of Grignard reagent (1.2-1.5 eq). The mixture is stirred at -20°C to 0°C for 1-6 hours, and column chromatography purification is carried out to give intermediate 1';

Condensation reaction 1: intermediate 1' (1.0 eq), halocarboxylic acid (0.6-3.0 eq), EDCI-HCl (1.0-6.0 eq), DMAP (0.05-0.5 eq), DIPEA (1.0-8.0 eq), and DCM are mixed sequentially, and stirred at room temperature overnight. The resultant is purified by column chromatography to obtain intermediate 2';

Condensation reaction 2: the intermediate 2' (1.0 eq) is treated with ammonium fluoride (5.0-20.0 eq) to remove TBS protection, then mixed with carboxylic acid (0.6-3.0 eq), EDCI-HCl (1.0-6.0 eq), DMAP (0.05-0.5 eq), DIPEA (1.0-8.0 eq), and DCM, and stirred overnight at room temperature. The resultant is purified by column chromatography to obtain intermediate 2;

Substitution reaction: intermediate 2 (1.0-5.0 eq), potassium carbonate (1.0-5.0 eq), amine compounds (1.0 eq), sodium iodide (1.0-3.0 eq), and acetonitrile are mixed sequentially, and stirred overnight at 20-100°C. The resultant is purified by column chromatography to obtain amino lipid.

### Lipid Nanoparticles

The present invention provides a lipid nanoparticle comprising any of the above amino lipid.

It will be understood that all of the above options and preferences for the amino lipid also apply to the lipid nanoparticles of the present invention that comprise amino lipids.

In some embodiments of the present invention, the lipid nanoparticle further comprises a steroid, a neutral lipid and/or a polymer-conjugated lipid.

### Steroid

A "steroid" is an organic compound with four rings arranged in a specific molecular configuration. It contains the following carbon skeleton:

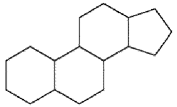

Steroids and neutral steroids include naturally occurring steroids and their analogues (such as amphiphilic lipid cholesterol hemisuccinate (CHEMS), composed of succinate linked by esterification to the β-hydroxyl of cholesterol, as a cholesterol derivative). Neutral steroids may be steroids that do not contain ionizable atoms or groups under physiological conditions, or may be zwitterionic steroids. In a preferred embodiment, neutral steroids do not contain atoms or groups that are ionizable under physiological conditions. In some preferred embodiments, the steroid or steroid analogue is cholesterol. The terms "steroid" and "neutral steroid" are used interchangeably herein.

### Neutral lipids

The "neutral lipids" of the present invention, also referred to as "auxiliary lipids", are preferably phospholipids or neutral phospholipids. As used herein, a "neutral phospholipid" is an amphiphilic compound composed of molecules typically having two hydrophobic fatty acid "tails" and a hydrophilic "head" containing a phosphate group. The phosphate group may be modified with simple organic molecules such as choline, ethanolamine, or serine. Phospholipids are abundant in nature. The term "phospholipid" or "neutral phospholipid" in this invention includes both natural and synthetic phospholipids.

### Polymer-conjugated lipid

The term "polymer-conjugated lipid" refers to molecules that simultaneously contain a lipid moiety and a polymer moiety. Preferably, the polymer-conjugated lipid is a PEGylated lipid or PEG-lipid. The terms "PEGylated lipid" or "PEG-lipid" refer to molecules that simultaneously contain a lipid moiety and a polyethylene glycol moiety. PEGylated lipids are known in the art and include PEG-DMG, etc.

In specific embodiments, the polymer-conjugated lipid has the chemical formula P-Y-L, where P is a hydrophilic polymer moiety, Y is an optional linker, and L is a lipid moiety.

Specifically, the hydrophilic polymer moiety P can be polyethylene glycol (PEG). In specific embodiments, the average molecular weight of the PEG moiety is between 1 kDa and 3 kDa, for example between 1.5 kDa and 2.5 kDa, between 1.7 kDa and 2.3 kDa, between 1.8 kDa and 2.2 kDa, between 1.9 kDa and 2.1 kDa, or 2 kDa. Therefore, PEG can be the PEG commonly referred to as "PEG 2000."

In another embodiment, the hydrophilic polymer moiety P in the polymer-conjugated lipid may also be a substantially hydrophilic polymer different from the aforementioned hydrophilic polymer moiety. That is, the hydrophilic polymer moiety P in the polymer-conjugated lipid may be based on poly(epoxypropane), poly(vinylpyrrolidone), poly(vinyl alcohol), poly-N-(2-hydroxypropyl)methacrylamide, hydroxyethyl starch modification (HESylation, according to PMID 24681396), PASylation (i.e., proline-alanine-serine), the XTEN method known in the art (i.e., PEG-based peptides), poly(sarcosine), or poly(vinyl acetate).

Specifically, the optional linker Y may be any useful spacer structure, such as those typically found in PEGylated lipids, including but not limited to those based on succinimide, amine, ether, ester, anhydride, aldehyde, ketone, amide, carbamate, or combinations thereof.

Specifically, the lipid moiety L may be derived from phospholipids, sphingolipids, or ceramides. As used herein, the term "derived from phospholipids or ceramides" includes free radicals of phospholipids and ceramides. An example is a polymer-conjugated lipid containing phosphatidylethanolamine moiety or phosphatidylglycerol moiety.

In a preferred embodiment, the polymer-conjugated lipid is a PEGylated lipid. The PEGylated lipid includes, but are not limited to, the following PEGylated lipids: PEGylated diacylglycerol lipid (PEG-DAG); PEGylated ceramide lipid (PEG-Cer); PEGylated phosphatidylethanolamine lipid (PEG-PE); PEGylated succinyl glycerol lipid (PEG-S-DAG); PEGylated dialkoxypropyl carbamate lipid; 1,2-dimyristyl-rac-glycero-3-methoxy polyethylene glycol ("PEG-DMG" or "DMG-PEG").

In a more preferred embodiment, the polymer-conjugated lipid is DMG-PEG2000.

Preferably, as used in the art, "DMG-PEG 2000" is considered to be a mixture of 1,2-DMG PEG2000 and 1,3-DMG PEG2000 in a ratio of about 97:3.

In some embodiments of the present invention, the molar ratio of amino lipid, steroid, neutral lipid, and polymer-conjugated lipid in the lipid nanoparticle is 30-70:30-65:0-30:0.2-5. More preferably, it is 30-60:35-60:0-20:0.3-3.

In a preferred embodiment of the present invention, in the lipid nanoparticle, the amino lipid is the aforementioned preferred amino lipid, the steroid is cholesterol, the neutral lipid is phospholipid, and the polymer-conjugated lipid is PEGylated lipid; the molar ratio of amino lipid, cholesterol, phospholipid, and PEGylated lipid is 40-50:40-45:10-15:0.5-2.

In a preferred embodiment, the PEGylated lipid in the lipid nanoparticle is DMG-PEG2000.

The lipid nanoparticle of the present invention is not limited to any specific form and should be interpreted as including any form produced when the amino lipid is combined with one or more of the other lipids, such as in an aqueous environment and/or in the presence of nucleic acid compounds. For example, liposomes, lipid complexes, lipoplexes and the like, fall within the scope of lipid nanoparticles.

The lipid nanoparticles of the present invention may be combined with at least one pharmaceutically acceptable carrier or excipient to form a pharmaceutical composition. Thus, the composition may be a dry composition, such as a powder or granule, or a solid unit, such as a lyophilized form or tablet. Alternatively, the composition may be in liquid form, and each excipient may be added independently in dissolved or dispersed form (e.g., suspension or emulsion). In a preferred embodiment, the composition is formulated as a sterile solid composition, such as a powder or lyophilized form, for reconstitution with an aqueous liquid carrier. This formulation is also preferred for compositions containing bioactive components, as further described below.

As used herein, "nanoparticles" are submicron particles of any structure or shape. Submicron particles can also be referred to as colloids or colloidal particles. Submicron particles can also be referred to as colloids or colloidal particles. Based on the materials, structure, or morphology of nanoparticles, nanoparticles can be classified into various types, such as nanocapsules, vesicles, liposomes, lipid nanoparticles, micelles, cross-linked micelles, lipoplexes, polymers, or hybrid or composite materials. Only a few examples of possible names for specific types of nanoparticles are mentioned. "Lipid nanoparticles" (LNPs) are nanoparticles formed from lipids, typically containing at least one amphiphilic film-forming lipid and optionally other lipids, and further optionally containing load materials such as nucleic acid compounds. As used herein, the terms "lipid nanoparticles" or "LNPs" include any subtype and morphology of nanoparticles formed from or co-formed with lipids, such as liposomes and lipoplexes.

As defined above, lipid nanoparticles include any type of nanoparticles formed or co-formed by lipids. Specifically, lipid nanoparticles may be co-formed by lipid combinations containing at least one amphiphilic vesicle-forming lipid. Liposomes and lipoplexes are examples of lipid nanoparticles.

Preferably, in some embodiments of the present invention, the lipid nanoparticles further contain bioactive components.

A bioactive component refers to any compound or material with biological activity, such that the compound or material may be used for the prevention, management, improvement, treatment or therapy of a disease or condition in a subject (e.g., an animal, particularly a human subject).

In some embodiments, the bioactive component is a nucleic acid compound selected from the group consisting of artificial mRNA, chemically modified or unmodified messenger RNA containing at least one coding sequence, self-replicating RNA, circular RNA, viral RNA, and replicon RNA; or any combination thereof. Preferably, the bioactive component is mRNA or an mRNA compound.

In some embodiments, the bioactive component is selected from small interfering RNA (siRNA), asymmetric interfering RNA (aiRNA), microRNA (miRNA), Dicer-substrate RNA (dsRNA), small hairpin RNA (shRNA), messenger RNA (mRNA), and mixtures thereof.

In embodiments of the present invention, the mRNA includes one or more of a stem-loop, chain-terminating nucleotides, a polyA sequence, a polyadenylation signal, and/or a 5' cap structure.

In embodiments of the present invention, the encapsulation efficiency of the bioactive component is at least 50-90%. More preferably, the encapsulation efficiency of the bioactive component is at least 60-80%.

In an embodiment of the present invention, the weight/weight ratio of the lipid component to the bioactive component in the lipid nanoparticle is about 10:1 to about 60:1. More preferably, the weight/weight ratio of the lipid component to the bioactive component is about 20:1.

In an embodiment of the present invention, the N:P ratio in the lipid nanoparticle is about 2:1 to about 30:1. More preferably, the N:P ratio is about 5.67:1.

In an embodiment of the present invention, the average size of the lipid nanoparticles is about 70 nm to about 100 nm.

In an embodiment of the present invention, the polydispersity index of the lipid nanoparticles is about 0.10 to about 0.20.

In embodiments of the present invention, the lipid nanoparticles have a zeta potential of about -10 mV to about +20 mV

In preferred embodiments, the bioactive component is complexed or combined with one or more lipids (e.g., amino lipids and/or neutral lipids) to form liposomes, lipid nanoparticles (LNPs), lipoplexes, and/or nanoliposomes. In the context, the terms "complexed" or "combined" refer to that the formation of a larger, non-covalently bonded complex or assembly of the bioactive component with one or more lipids in a substantially stable manner.

### Use

The present invention provides a method for treating or preventing infectious diseases, cancer, tumour diseases, genetic diseases, allergy, toxicity, and autoimmune diseases using the aforementioned amino lipid or lipid nanoparticle or pharmaceutical composition.

Accordingly, the present invention provides the use of the aforementioned amino lipid or lipid nanoparticle or pharmaceutical composition in the preparation of a medicament for preventing or treating infectious diseases, cancer, tumour diseases, genetic diseases, allergy, toxicity, and autoimmune diseases.

The infectious diseases include infectious diseases of virus, bacteria, or protozoan. The viruses include, but are not limited to, SARS coronavirus 2 (SARS-CoV-2), nCoV-2019 coronavirus, SARS coronavirus (SARS-CoV), bunyaviruses, cytomegalovirus (CMV), dengue viruses (DEN-1, DEN-2, DEN-3, and DEN-4), Ebola virus, flavivirus, hepatitis B virus (HBV), herpes simplex virus (HSV), human immunodeficiency virus (HIV), human metapneumovirus (hMPV), human papillomavirus (HPV), human parainfluenza virus (HPIV), influenza virus, enteropathogenic Escherichia coli, Lassa virus (LASV), Middle East Respiratory Syndrome Coronavirus (MERS-CoV), Mycobacterium tuberculosis, Nipah virus, Norovirus, Rabies virus, Respiratory Syncytial Virus (RSV), Rhinovirus, Rotavirus, Vaccinia virus, Yellow Fever virus, and Zika virus.

The cancers include lung cancer, gastric cancer, liver cancer, esophageal cancer, colon cancer, pancreatic cancer, brain cancer, lymphoma, leukaemia, and prostate cancer.

The present invention also provides a method for gene therapy, genetic vaccination, antisense therapy, delivery of nucleic acids, or treatment through RNA interference, by using the aforementioned amino lipid or lipid nanoparticle or pharmaceutical composition.

Accordingly, the present invention also provides the use of the aforementioned amino lipid or lipid nanoparticle in the preparation of a medicament for gene therapy, genetic vaccination, antisense therapy, delivery of nucleic acids, or treatment through RNA interference.

The present invention also provides a method for delivering a medicament to a subject, comprising: administering the medicament formulated in the aforementioned lipid nanoparticle to the subject.

When administering the medicament, the administration method is determined based on the formulation of the medicament. The formulation of the medicament is related to the excipients and/or carriers. General routes for systemic administration include: transdermal, oral, and parenteral routes, including subcutaneous, intravenous, intramuscular, intra-arterial, intradermal, and intraperitoneal injections, and/or intranasal administration routes. Local administration routes generally include intradermal, transdermal, subcutaneous, or intramuscular injection; or intralesional, intracranial, pulmonary, intracardiac, intratumoural, or sublingual injection. When the medicament is in vaccine form, the preferred administration routes are intramuscular and intradermal injection.

The following examples are provided to illustrate the present invention but are not intended to limit the scope of the invention.

Unless otherwise specified, the techniques or conditions used in the examples are those described in the literature of the field or as described in the product manuals. Unless otherwise specified, the reagents or instruments used are conventional products available through regular channels.

For illustrative purposes, the following examples show the general methods for preparing the compounds provided by the present invention. For more detailed descriptions of each reaction step, please refer to the following examples. Those skilled in the art will understand that other synthetic routes may be used to synthesize the compounds of the present invention. Although specific raw materials and reagents are described in the scheme and discussed below, other raw materials and reagents may be readily used instead to provide various derivatives and/or reaction conditions. Furthermore, in combination with the disclosure of the following examples, many compounds prepared by the methods described below may be further modified using conventional chemical methods known to those skilled in the art to obtain other compounds within the scope of the present invention.

The abbreviations used correspond to the following substances:
Py: Pyridine
EDCI: 1-Ethyl-(3-dimethylaminopropyl)carbodiimide hydrochloride
DMAP: 4-Dimethylaminopyridine
DIPEA: Diisopropylethylamine
DCM: Dichloromethane
MeCN: Acetonitrile
DSPC: Distearoylphosphatidylcholine
DMG-PEG2000: 1,2-dimyristyl-rac-glycero-3-methoxy polyethylene glycol 2000.

### Preparation of amino lipid compounds

### Example 1 Synthesis of amino lipid E8LA12B6O3

### Step 1: Synthesis of E8LA12:

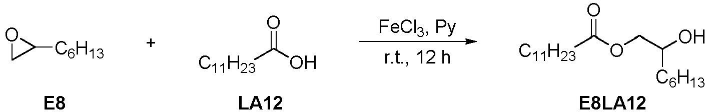

FeCl₃ (16.2 mg, 2.5 mol%), Py (4 mg, 1.25 mmol%), lauric acid (200.32 g/mol, 800 mg, 4.0 mmol) and 1,2-epoxyoctane (128.22 g/mol, 1.02 g, 8 mmol) were added in sequence into a 25 mL reaction tube. The reaction solution was stirred at room temperature overnight. After the reaction was completed, 1.2 g of intermediate **E8LA12**(colorless oily liquid) was obtained by purification via column chromatography separation, with a yield of 91%.

### Step 2: Synthesis of E8LA12B6:

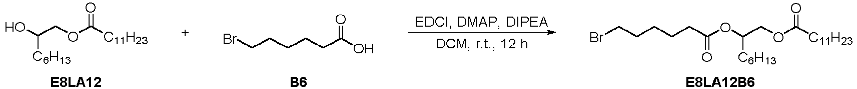

**E8LA12** (328.54 g/mol, 1.2 g, 3.65 mmol), 6-bromohexanoic acid (195.06 g/mol, 855 mg, 4.38 mmol), EDCI(191.70 g/mol, 2.8 g, 14.6 mmol), DMAP(45 mg, 0.365 mmol), DIPEA(2.83 g, 21.9 mmol), and DCM(10 mL) were added in sequence into a 25 mL reaction tube. The reaction solution was stirred at room temperature overnight. After the reaction was completed, 1.65 g of intermediate **E8LA12B6**(colorless oily liquid) was obtained by purification via column chromatography separation, with a yield of 89%.

### Step 3: Synthesis of E8LA12B6O3:

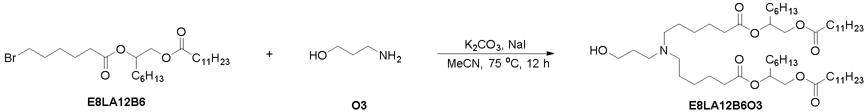

**E8LA12B6** (505.58 g/mol, 1.65 g, 3.26 mmol), potassium carbonate (138.21 g/mol, 902 mg, 6.52 mmol), sodium iodide (149.89 g/mol, 489 mg, 3.26 mmol), aminopropanol (75.11 g/mol, 113 mg , 1.5 mmol) and acetonitrile (10 mL) were added in sequence into a 25 mL reaction tube. The reaction solution was stirred at 75°C overnight. After the reaction was completed, 0.94 g of **E8LA12B6O3** (colorless oily liquid) was obtained by purification via column chromatography separation, with a yield of 68%.

¹H NMR (400 MHz, CDCl₃): *δ* 5.08-5.03 (m, 2H), 4.22 (dd, *J*₁ = 11.6 Hz, *J*₂ = 3.6 Hz, 2H), 4.02 (dd, *J*₁ = 12.0 Hz, *J*₂ = 6.4 Hz, 2H), 3.82 (t, *J =* 5.6 Hz, 2H), 2.96 (t, *J* = 7.6 Hz, 2H), 2.77 (t, *J =* 6.0 Hz, 4H), 2.34-2.28 (m, 8H), 1.89-1.88 (m, 2H), 1.72-1.56 (m, 12H), 1.40-1.25 (m, 56H), 0.87 (t, *J* = 7.0 Hz, 12H). ¹³C NMR (100 MHz, CDCl₃): *δ* 173.1, 70.7, 65.5, 59.0, 58.5, 57.2, 34.2, 33.9, 31.9, 31.8, 30.7, 30.3, 29.6, 29.3, 29.0, 28.0, 26.7, 25.0, 25.3, 24.7, 22.7, 14.1. ESI-MS C₅₅H₁₀₆NO₉⁺ [M+H]⁺ calcd. 924.7862, found 924.7850.

### Example 2 Synthesis of amino lipid E12LA6B6O3

### Step 1: Synthesis of E12LA6:

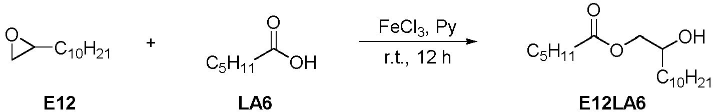

FeCl₃ (16.2 mg, 2.5 mol%), Py (4 mg, 1.25 mmol%), n-hexanoic acid (116.1600 g/mol, 464 mg, 4.0 mmol) and 1,2-epoxydodecane (184.18 g/mol, 1.47 g, 8 mmol) were added in sequence into a 25 mL reaction tube. The reaction solution was stirred at room temperature overnight. After the reaction was completed, 1.02 g of intermediate **E12LA6** (colorless oily liquid) was obtained by purification via column chromatography separation with a yield of 85%.

### Step 2: Synthesis of E12LA6B6:

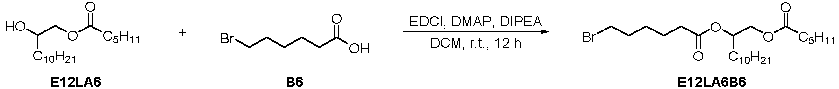

**E12LA6** (300.48 g/mol, 901 mg, 3.0 mmol), 6-bromohexanoic acid (195.06 g/mol, 703 mg, 3.6 mmol), EDCI (191.70 g/mol, 2.3 g, 12.0 mmol), DMAP (37 mg, 0.3 mmol), DIPEA (2.33 g, 18 mmol), and DCM (10 mL) were added in sequence into a 25 mL reaction tube. The reaction solution was stirred at room temperature overnight. After the reaction was completed, 1.08 g of intermediate **E12LA6B6** (colorless oily liquid) was obtained by purification via column chromatography separation, with a yield of 76%.

### Step 3: Synthesis of E12LA6B6O3:

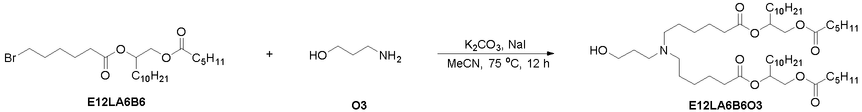

**E12LA6B6** (477.52 g/mol, 956 mg, 2.0 mmol), potassium carbonate (138.21 g/mol, 221 mg, 1.6 mmol), sodium iodide (149.89 g/mol, 120 mg, 0.8 mmol), aminopropanol (75.11 g/mol, 60 mg, 0.8 mmol) and acetonitrile (5 mL) were added in sequence into a 25 mL reaction tube. The reaction solution was stirred at 75°C overnight. After the reaction was completed, 458 mg **E12LA6B6O3** (colorless oily liquid) was obtained by purification via column chromatography separation, with a yield of 66%.

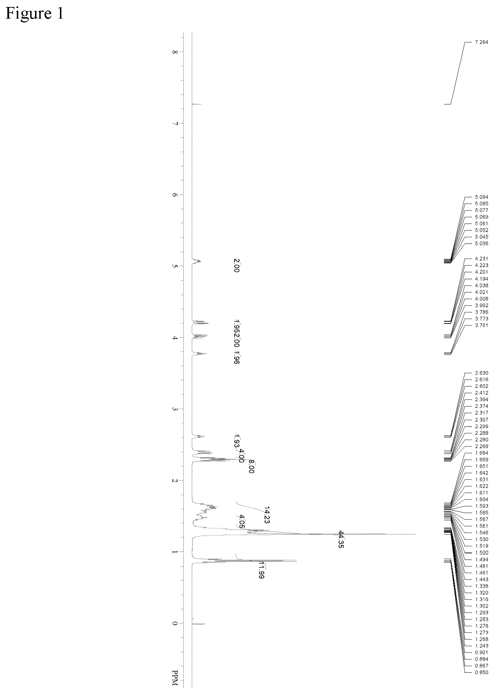

¹H NMR (400 MHz, CDCl₃): *δ* 5.08-5.06 (m, 2H), 4.22 (dd, *J*₁ = 12.0 Hz, *J*₂ = 3.6 Hz, 2H), 4.02 (dd, *J*₁ = 11.6 Hz, *J*₂ = 6.4 Hz, 2H), 3.79 (t, *J* = 5.2 Hz, 2H), 2.73-2.70 (m, 2H), 2.52-2.48 (m, 4H), 2.33-2.28 (m, 8H), 1.74-1.50 (m, 14H), 1.42-1.25 (m, 48H), 0.91-0.86 (m, 12H). (As shown in Figure 1) ESI-MS C₅₁H₉₈NO₉⁺ [M+H]⁺ calcd. 868.7236, found 868.7234.

### Example 3 Synthesis of amino lipid E8LA8B6O3

The amino lipid **E8LA8B6O3** can be prepared according to the same method as example 1 except replacing lauric acid in Example 1 with caprylic acid, and its structural formula is as follows:

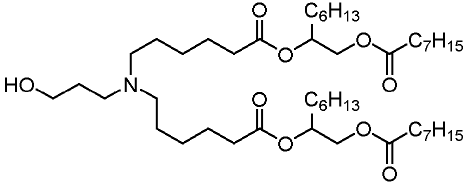

¹H NMR (400 MHz, CDCl₃): *δ* 5.08-5.02 (m, 2H), 4.22 (dd, *J*₁ = 12.0 Hz, *J*₂ = 3.6 Hz, 2H), 4.01 (dd, *J*₁ = 11.6 Hz, *J*₂ = 6.0 Hz, 2H), 3.81 (t, *J* = 5.2 Hz, 2H), 2.98 (t, *J* = 6.0 Hz, 2H), 2.79 (t, *J =* 7.6 Hz, 4H), 2.34-2.27 (m, 8H), 1.92-1.86 (m, 2H), 1.73-1.56 (m, 12H), 1.40-1.26 (m, 40H), 0.88-0.85 (m, 12H). ESI-MS C₄₇H₉₀NO₉⁺ [M+H]⁺ calcd. 812.6610, found 812.6598.

### Example 4 Synthesis of amino lipid E8LA10B6O3

The amino lipid E8LA10B603 can be prepared according to the same method as example 1 except replacing lauric acid in Example 1 with capric acid, and its structural formula is as follows:

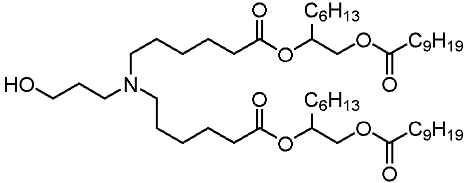

¹H NMR (400 MHz, CDCl₃) : δ 0.85-0.89 (m, 12H), 1.25-1.40 (m, 48H), 1.56-1.72 (m, 12H), 1.88-1.90 (m, 2H), 2.28-2.33 (m, 8H), 2.77-2.79 (m, 4H), 2.96-2.98 (m, 2H), 3.79-3.80 (m, 2H), 4.01-4.05 (m, 2H), 4.21-4.24 (m, 2H), 5.03-5.08 (m, 2H). ESI-MSC₅₁H₉₈NO₉⁺ [M+H]⁺ calcd. 868.7, found 868.7.

### Example 5 Synthesis of amino lipid E10LA6B6O3

The amino lipid E10LA6B603 can be prepared according to the same method as example 2 except replacing 1,2-epoxydodecane in Example 2 with 1,2-epoxydecane, and its structural formula is as follows:

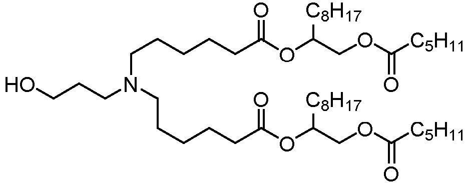

¹H NMR (400 MHz, CDCl₃): *δ* 5.08-5.02 (m, 2H), 4.22 (dd, *J*₁ = 12.0 Hz, *J*₂ = 3.2 Hz, 2H), 4.01 (dd, *J*₁ = 11.6 Hz, *J*₂ = 6.4 Hz, 2H), 3.79 (t, *J* = 5.2 Hz, 2H), 2.82 (t, *J* = 5.6 Hz, 2H), 2.62 (t, *J=* 8.0 Hz, 4H), 2.36-2.27 (m, 8H), 1.81-1.76 (m, 2H), 1.68-1.53 (m, 12H), 1.37-1.24 (m, 40H), 0.90-0.84 (m, 12H). ESI-MS C₄₇H₉₀NO₉⁺ [M+H]⁺ calcd. 812.6610, found 812.6634.

### Example 6 Synthesis of amino lipid E10LA8B6O3

The amino lipid E10LA8B603 can be prepared according to the same method as example 1 except replacing1,2-epoxyoctane in Example 1 with 1,2-epoxydecane and by replacing lauric acid in Example 1 with n-octanoic acid, and its structural formula is as follows:

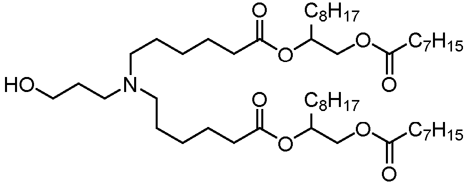

¹H NMR (400 MHz, CDCl₃) : *δ* 0.84-0.89 (m, 12H), 1.26-1.41 (m, 48H), 1.56-1.73 (m, 12H), 1.89-1.91 (m, 2H), 2.26-2.32 (m, 8H), 2.78-2.80 (m, 4H), 2.96-2.98 (m, 2H), 3.80-3.81 (m, 2H), 4.01-4.04 (m, 2H), 4.20-4.24 (m, 2H), 5.03-5.07 (m, 2H). ESI-MSC₅₁H₉₈NO₉⁺ [M+H]⁺ calcd. 868.7, found 868.7.

### Example 7 Synthesis of amino lipid E10LA10B6O3

The amino lipid E10LA10B603 can be prepared according to the same method as example 1 except replacing 1,2-epoxyoctane in Example 1 with 1,2-epoxydecane and by replacing lauric acid in Example 1 with capric acid, and its structural formula is as follows:

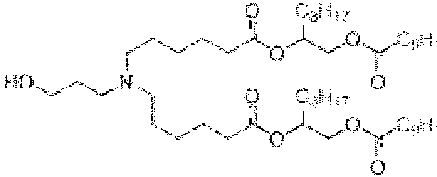

¹H NMR (400 MHz, CDCl₃) : δ 0.86-0.92 (m, 12H), 1.25-1.40 (m, 56H), 1.56-1.71 (m, 12H), 1.87-1.89 (m, 2H), 2.27-2.32 (m, 8H), 2.77-2.80 (m, 4H), 2.97-2.99 (m, 2H), 3.79-3.80, (m, 2H), 4.00-4.04 (m, 2H), 4.21-4.25 (m, 2H), 5.02-5.07 (m, 2H). ESI-MSC₅₅H₁₀₆NO₉⁺ [M+H]⁺ calcd. 924.8, found 924.9.

### Example 8 Synthesis of amino lipid E12LA8B6O3

The amino lipid E12LA8B603 can be prepared according to the same method as example 2 except replacing n-hexanoic acid in Example 2 with n-octanoic acid, and its structural formula is as follows:

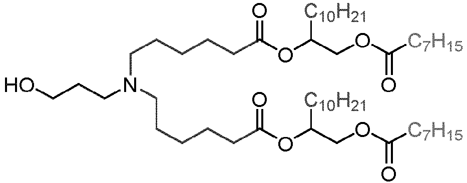

¹H NMR (400 MHz, CDCl₃): *δ* 5.07-5.04 (m, 2H), 4.23 (dd, *J*₁ = 12.0 Hz, *J*₂ = 3.6 Hz, 2H), 4.01 (dd, *J*₁ = 12.0 Hz, *J*₂ = 6.4 Hz, 2H), 3.80 (t, *J =* 5.2 Hz, 2H), 2.86 (t, *J =* 5.6 Hz, 2H), 2.66 (t, *J =* 7.6 Hz, 4H), 2.33-2.28 (m, 8H), 1.83-1.80 (m, 2H), 1.69-1.56 (m, 12H), 1.38-1.25 (m, 56H), 0.89-0.85 (m, 12H). ESI-MS C₅₅H₁₀₆NO₉⁺ [M+H]⁺ calcd. 924.7862, found 924.7891.

### Example 9 Synthesis of compound E12LA12B6O3

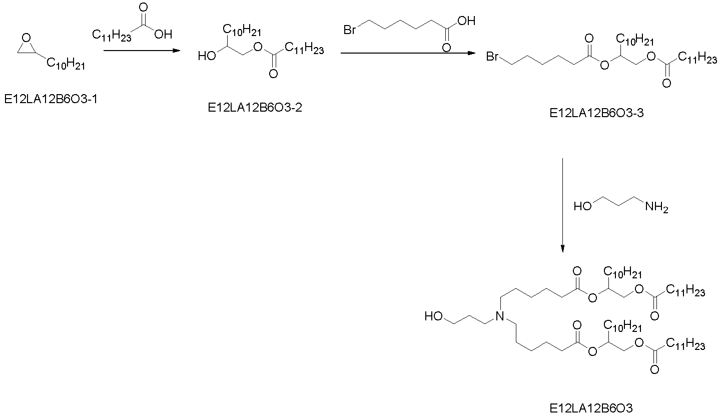

### Step 1: Synthesis of compound E12LA12B6O3-2

Ferric chloride (16.2 mg, 0.1 mmol, 0.025 eq), pyridine(4 mg, 0.05 mmol, 0.0125 eq), lauric acid( 800 mg, 4.0 mmol, 1.0 eq) and 1,2-epoxydodecane (884 mg, 4.8 mmol, 1.2 eq) were added in sequence into a 25 mL reaction tube. The reaction solution was stirred at room temperature overnight. After the reaction was completed, the reaction solution was concentrated and purified using a flash column chromatography system (n-heptane: ethyl acetate = 50: 1 to 10: 1) to obtain the compound **E12LA12B6O3-2(1.2** g, 86.0%). ESI-MS C₂₄H₄₉O₃⁺ [M+H]⁺ calcd. 385.4, found 385.4.

### Step 2: Synthesis of compound E12LA12B6O3-3

**E12LA12B6O3-2** (1.2 g, 3.65 mmol, 1.0 eq), 6-bromohexanoic acid (973 mg, 4.38 mmol, 1.2 eq), 1-(3-dimethylaminopropyl)-3-ethylcarbodiimide hydrochloride (2.8 g, 14.6 mmol, 4.0 eq) , 4-dimethylaminopyridine (45 mg , 0.365 mmol, 0.1 eq) and N,N-diisopropylethylamine (2.83 g, 21.9 mmol, 6.0 eq) and 20 mL dichloromethane were added in sequence into a 25 mL reaction tube. The reaction solution was stirred at room temperature overnight. After the reaction was completed, the reaction solution was concentrated and purified using a flash column chromatography system (n-heptane:ethyl acetate =100:1 to 20:1) to obtain the compound **E12LA12B6O3-3** (1.65 g, 92.0%). ESI-MS C₃₀H₅₈BrO₄⁺ [M+H]⁺ calcd. 561.4, found 561.4.

### Step 3: Synthesis of E12LA12B6O3

**E12LA12B6O3-3** (1.65 g, 3.36 mmol, 2.2 eq), potassium carbonate ( 464 mg, 3.36 mmol, 2.2 eq), sodium iodide( 150 mg, 1.0 mmol, 0.67 eq), 3-aminopropanol (113 mg, 1.5 mmol), and 10 mL acetonitrile were added in sequence into a 25 mL reaction tube. The reaction solution was stirred at 70-80°C overnight. After the reaction was completed, 0.94 g E12LA12B603 (0.94 g, 68%) was obtained via column chromatography separation.

¹H NMR (400 MHz, CDCl₃) : δ 0.87-0.91 (m, 12H), 1.24-1.38 (m, 68H), 1.42-1.58 (m, 4H), 1.55-1.69 (m, 14H), 2.29-2.34 (m, 8H), 2.38-2.42 (m, 4H), 2.61-2.64 (m, 2H), 3.78-3.81, (m, 2H), 4.01-4.06 (m, 2H), 4.20-4.24 (m, 2H), 5.04-5.10 (m, 2H). ESI-MS C₆₃H₁₂₂NO₉⁺ [M+H]⁺ calcd. 1036.9, found 1037.0.

### Example 10 Synthesis of amino lipid E8CA5B6O3

The amino lipid E8CA5B6O3 can be prepared according to the same method as example 9 except replacing 1,2-epoxydodecane in Example 9 with 1,2-epoxyoctane and replacing lauric acid in Example 9 with 2-pentyl-heptanoic acid, and its structural formula is as follows:

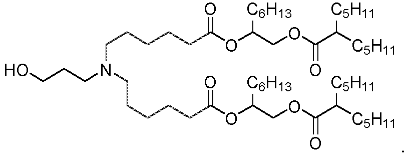

¹H NMR (400 MHz, CDCl₃) : δ 0.85-0.90 (m, 18H), 1.25-1.37 (m, 48H), 1.40-1.70 (m, 18H), 2.25-2.36 (m, 6H), 2.44-2.48 (m, 4H), 2.66-2.68 (m, 2H), 3.77-3.79, (m, 2H), 4.00-4.05 (m, 2H), 4.21-4.25 (m, 2H), 5.03-5.08 (m, 2H). ESI-MS C₅₅H₁₀₆NO₉⁺ [M+H]⁺ calcd. 924.8, found 924.8.

### Example 11 Synthesis of amino lipid E12LA6B6O9

The amino lipid E12LA6B609 can be prepared according to the same method as example 2 except replacing the 3-aminopropanol in Example 2 with n-pentylamine, and its structural formula is as follows:

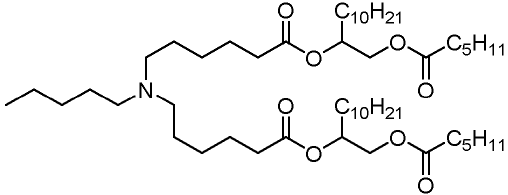

¹H NMR (400 MHz, CDCl₃) : δ 0.85-0.90 (m, 15H), 1.24-1.36 (m, 54H), 1.53-1.74 (m, 16H), 2.27-2.33 (m, 8H), 2.83 (s, 6H), 3.98-4.03 (m, 2H), 4.21-4.25 (m, 2H), 5.02-5.07 (m, 2H). ESI-MS C₅₃H₁₀₂NO₈⁺ [M+H]⁺ calcd. 880.8, found 880.8

### Example 12 Synthesis of amino lipid E12LA6B6O10

The amino lipid E12LA6B6010 can be prepared according to the same method as example 2 except replacing 3-aminopropanol in Example 2 with trans-p-aminocyclohexanol, and its structural formula is as follows:

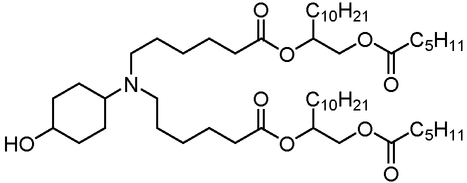

¹H NMR (400 MHz, CDCl₃) : δ 0.84-0.90 (m, 12H), 1.24-1.31 (m, 54H), 1.53-1.65 (m, 14H), 1.99-2.01 (m, 2H), 2.26-2.31 (m, 8H), 2.42-2.46 (m, 4H), 3.99-4.03 (m, 2H), 4.19-4.22 (m, 2H), 5.03-5.08 (m, 2H). ESI-MS C₅₄H₁₀₂NO₉⁺ [M+H]⁺ calcd. 908.8, found 908.7.

### Example 13 Synthesis of amino lipid E12LA6B6O12

The amino lipid E12LA6B6012 can be prepared according to the same method as example 2 except replacing 3-aminopropanol in Example 2 with 3-dimethylaminopropylamine, and its structural formula is as follows:

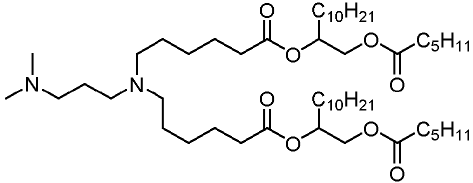

¹H NMR (400 MHz, CDCl₃) : *δ* 0.86-0.94 (m, 12H), 1.26-1.35 (m, 42H), 1.41-1.48 (m, 6H), 1.55-1.72 (m, 18H), 2.29-2.36 (m, 8H), 2.70-2.76 (m, 2H), 3.37-3.41 (m, 4H), 3.99-4.04 (m, 2H), 4.24-4.34 (m, 6H), 5.00-5.06 (m, 2H). ESI-MS C₅₃H₁₀₃N₂O₈⁺ [M+H]⁺ calcd. 895.8, found 895.8.

### Example 14 Synthesis of amino lipid E12LA6B6O13

The amino lipid E12LA6B6013 can be prepared according to the same method as example 2 except replacing 3-aminopropanol in Example 2 with 1-(2-aminoethyl)piperidine, and its structural formula is as follows:

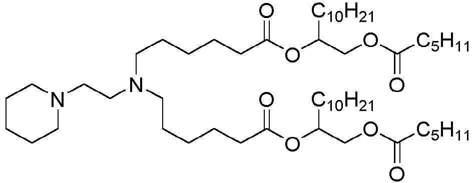

¹H NMR (400 MHz, CDCl₃) : δ 0.82-0.92 (m, 12H), 1.24-1.36 (m, 50H), 1.44-1.69 (m, 18H), 2.27-2.31 (m, 8H), 2.47-2.50 (m, 4H), 2.57-2.61 (m, 4H), 2.72-2.74 (m, 2H), 3.99-4.04 (m, 2H), 4.19-4.23 (m, 2H), 5.03-5.08 (m, 2H). ESI-MS C₅₅H₁₀₅N₂O₈⁺ [M+H]⁺ calcd. 921.8, found 921.8.

### Example 15 Synthesis of amino lipid E12LA6B6O15

The amino lipid E12LA6B6015 can be prepared according to the same method as example 2 except replacing 3-aminopropanol in Example 2 with N-(2-aminoethyl)-4-hydroxypiperidine, and its structural formula is as follows:

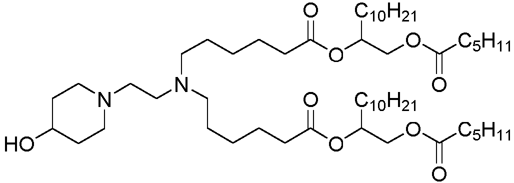

¹H NMR (400 MHz, CDCl₃) : δ 0.86-0.90 (m, 12H), 1.26-1.37 (m, 50H), 1.48-1.67 (m, 16H), 2.28-2.33 (m, 8H), 2.54-2.61 (m, 6H), 2.84-2.89 (m, 4H), 3.76 (s, 1H). 4.00-4.07 (m, 2H), 4.20-4.24 (m, 2H), 5.04-5.09 (m, 2H). ESI-MS C₅₅H₁₀₅N₂O₉⁺ [M+H]⁺ calcd. 937.8, found 937.9.

### Example 16 Synthesis of amino lipid E12LA12B6O30

The amino lipid E12LA12B6030 can be prepared according to the same method as example 2 except replacing 3-aminopropanol in Example 2 with β-alanine, and its structural formula is as follows:

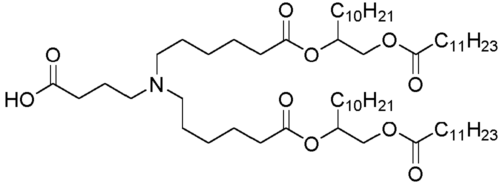

¹H NMR (400 MHz, CDCl₃) : δ 0.86-0.92 (m, 12H), 1.24-1.37 (m, 68H), 1.44-1.60 (m, 4H), 1.54-1.70 (m, 14H), 2.29-2.44 (m, 12H), 2.61-2.64 (m, 2H), 3.78-3.81, (m, 2H), 4.01-4.05 (m, 2H), 4.21-4.24 (m, 2H), 5.02-5.08 (m, 2H). ESI-MS C₆₄H₁₂₂NO₁₀⁺ [M+H]⁺ calcd. 1064.9, found 1065.0.

### Example 17 Synthesis of amino lipid E12LA6B6O31

The amino lipid E12LA12B6031 can be prepared according to the same method as example 2 except replacing 3-aminopropanol in Example 2 with 4-aminobutyronitrile, and its structural formula is as follows:

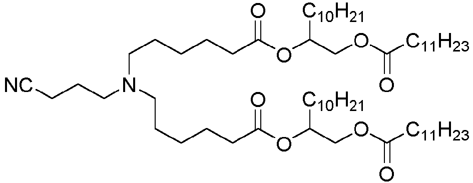

¹H NMR (400 MHz, CDCl₃) : δ 0.86-0.91 (m, 12H), 1.25-1.39 (m, 68H), 1.42-1.60 (m, 4H), 1.58-1.72 (m, 12H), 1.78-1.82 (m, 2H), 2.31-2.42 (m, 12H), 2.60-2.64 (m, 2H), 3.79-3.82, (m, 2H), 4.00-4.04 (m, 2H), 4.18-4.22 (m, 2H), 5.02-5.09 (m, 2H). ESI-MS C₆₄H₁₂₁N₂O₈⁺ [M+H]⁺ calcd. 1045.9, found 1045.9.

### Example 18 Synthesis of amino lipid K3LA6B6O3

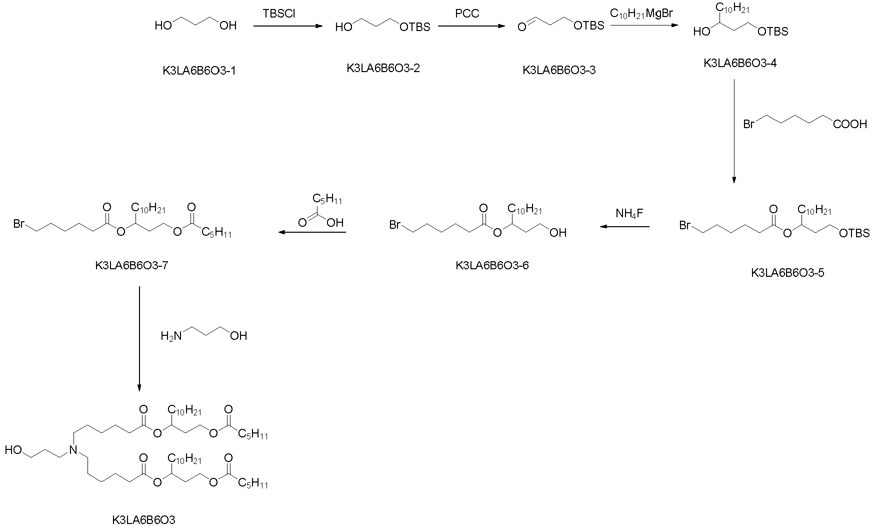

### Step 1: Synthesis of compound K3LA6B6O3-2

Imidazole (6.54 g, 96 mmol, 1.2 eq ) was added to a 250 mL round-bottom flask, and the flask was filled with nitrogen by evacuation. Dichloromethane (60 mL) and N,N-dimethylformamide (30 mL) and 1,3-propylene glycol (6.09 g, 80 mmol, 1.0 eq) were added in sequence, and the mixture was stirred at 0°C for 10 minutes. Tert-butyldimethylsilyl chloride (12.06 g, 80 mmol, 1.0 eq) was dissolved in dichloromethane (60 mL) and added dropwise to the mixture. The resulting mixture was stirred at room temperature for 4 hours. The reaction mixture was washed with water three times, dried over anhydrous sodium sulfate, concentrated, and purified using a flash column chromatography system (n-heptane: ethyl acetate = 100: 1 to 10: 1) to obtain a compound K3LA6B6O3-2(10.96 g, 72%), ESI-MS C₉H₂₃O₂Si⁺ [M+H]⁺ calcd. 191.1, found 191.1.

### Step 2: Synthesis of compound K3LA6B6O3-3

A solution of pyridinium chlorochromate (6.09 g, 80 mmol, 1.0 eq) and compound **K3LA6B6O3-2** (10.96 g, 57.6 mmol, 1.0 eq) in dichloromethane (100 mL) was stirred at room temperature for 5 hours. The reaction mixture was filtered and then the filtrate was concentrated and purified using a flash column chromatography system (n-heptane: ethyl acetate = 100:1 to 20:1) to obtain a compound K3LA6B6O3-3 (8.69 g, 80%), ESI-MS C₉H₂₁O₂Si⁺ [M+H]⁺ calcd. 189.1, found 189.1.

### Step 3: Synthesis of compound K3LA6B6O3-4

A 500 mL round-bottom flask was taken and filled with nitrogen by evacuation. The compound K3LA6B6O3-3(8.69 g, 46.14 mmol, 1.0 eq) and anhydrous tetrahydrofuran (150 mL) were added. The mixture was stirred at -20°C for 10 minutes. 70 mL of decylmagnesium bromide solution (1 mol/L anhydrous tetrahydrofuran solution, 1.5 eq) was added dropwise to the mixture. The mixture was stirred at -20°C to 0°C for 3 hours, 50 mL of saturated ammonium chloride solution was added for quenching. The resultant solution was extracted three times with ethyl acetate, washed once with saturated brine, dried over anhydrous sodium sulfate, concentrated, and purified using a flash column chromatography system (n-heptane:ethyl acetate=100:1 to 20:1) to obtain a compound **K3LA6B6O3-4** (9.93g, 65%), ESI-MS C₁₉H₄₃O₂Si⁺ [M+H]⁺ calcd. 331.3, found 331.4.

### Step 4: Synthesis of compound K3LA6B6O3-5

A solution of **K3LA6B6O3-4** (9.93 g, 30 mmol), 6-bromohexanoic acid (7.02 g, 36 mmol, 1.2 eq), 1-(3-dimethylaminopropyl)-3-ethylcarbodiimide hydrochloride (11.50 g, 60 mmol, 2.0 eq), N,N-diisopropylethylamine(15.51 g, 120 mmol, 4.0 eq), and 4-dimethylaminopyridine (0.37 g, 3 mmol, 0.1 eq) in dichloromethane (100 mL) was stirred at room temperature overnight. TLC showed that the reaction was completed. The reaction solution was extracted three times with dichloromethane, washed once with saturated brine, dried over anhydrous sodium sulfate, concentrated, and purified using a flash column chromatography system (n-heptane:ethyl acetate =100:1 to 20:1) to obtain a compound **K3LA6B6O3-5**(12.79 g, 84%), ESI-MS C₂₅H₅₂BrO₃Si⁺ [M+H]⁺ calcd. 507.3, found507.2.

### Step 5: Synthesis of compound K3LA6B6O3-6

A solution of **K3LA6B6O3-5** (12.79 g, 25.2 mmol, 1.0 eq), ammonium fluoride (9.33 g, 252 mmol, 10.0 eq), and methanol(75 mL) was refluxed at 65°C and stirred for 3 hours. TLC showed that the reaction was completed. The reaction solution was extracted three times with ethyl acetate, washed once with saturated brine, dried over anhydrous sodium sulfate, concentrated, and purified using a flash column chromatography system (n-heptane:ethyl acetate =50:1 to 5:1) to obtain a compound **K3LA6B6O3-6**(8.**72** g, 88%), ESI-MS C₁₉H₃₈BrO₃⁺ [M+H]⁺ calcd. 393.2, found393.2.

### Step 6: Synthesis of compound K3LA6B6O3-7

A solution of **K3LA6B6O3-6** (8.72 g, 22.2 mmol), hexanoic acid(3.09 g, 26.64 mmol, 1.2 eq), 1-(3-dimethylaminopropyl)-3-ethylcarbodiimide hydrochloride(8.51 g, 44.4 mmol, 2.0 eq), N,N-diisopropylethylamine(11.48 g, 88.8 mmol, 4.0 eq), and 4-dimethylaminopyridine(0.27 g, 2.2 mmol, 0.1 eq) in dichloromethane (50 mL) was stirred at room temperature overnight. TLC showed that the reaction was completed. The reaction solution was extracted three times with dichloromethane, washed once with saturated brine, dried over anhydrous sodium sulfate, concentrated, and purified using a flash column chromatography system (n-heptane:ethyl acetate =100:1 to 20:1) to obtain a compound K3LA6B6O3-7 (7.86 g, 72%), ESI-MS C₂₅H₄₈BrO₄⁺ [M+H]⁺ calcd. 491.3, found 491.2.

### Step 7: Synthesis of compound K3LA6B6O3

A solution of compound **K3LA6B6O3-7** (7.86 g, 15.99 mmol, 3.0 eq), 3-aminopropanol (0.40 g, 5.33 mmol, 3.0 eq), potassium carbonate (1.47 g, 10.66 mmol, 2.0 eq), sodium iodide (0.80 g, 5.33 mmol, 1.0 eq) in acetonitrile (10 mL) was stirred at 75°C overnight. After the reaction solution was concentrated, it was purified using a flash column chromatography system (dichloromethane:methanol =100:1 to 20:1 ) to obtain a compound K3LA6B6O3-7(3.58 g, 75%), ¹H NMR (400 MHz, CDCl₃) : δ 0.85-0.90 (m, 12H), 1.24-1.36 (m, 48H), 1.56-1.73 (m, 18H), 2.25-2.29 (m, 8H), 2.48-2.52 (m, 4H), 2.70-2.72 (m, 2H), 3.76-3.79 (m, 2H), 4.03-4.11 (m, 4H), 4.93-4.96 (m, 2H). ESI-MS C₅₃H₁₀₂NO₉⁺ [M+H]⁺ calcd. 896.8, found 896.9.

### Example 19 Synthesis of amino lipid K4LA6B6O3

The amino lipid **K4LA6B6O3** can be prepared according to the same method as example 18 except replacing the 1,3-propylene glycol in Example 18 with 1,4-butylene glycol, and its structural formula is as follows:

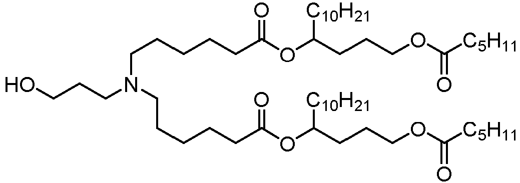

¹H NMR (400 MHz, CDCl₃) : *δ* 0.85-0.91 (m, 12H), 1.26-1.41 (m, 48H), 1.56-1.72 (m, 18H), 2.27-2.33 (m, 8H), 2.81-2.84 (m, 4H), 3.00-3.04 (m, 2H), 3.81-3.84 (m, 2H), 4.04-4.07 (m, 4H), 4.87-4.90 (m, 2H). ESI-MS C₅₅H₁₀₆NO₉⁺ [M+H]⁺ calcd. 924.8, found 924.8.

### Example 20 Synthesis of amino lipid K5LA6B6O3

The amino lipid **K5LA6B6O3** can be prepared according to the same method as example 18 except replacing the 1,3-propylene glycol in Example 18 with 1,5-pentanediol, and its structural formula is as follows:

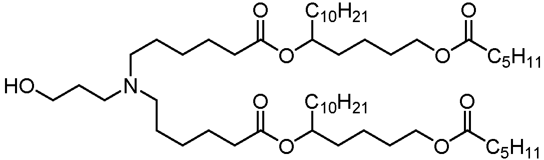

¹H NMR (400 MHz, CDCl₃) : δ 0.86-0.91 (m, 12H), 1.26-1.41 (m, 54H), 1.50-1.67 (m, 18H), 2.27-2.34 (m, 8H), 2.91-2.96 (m, 4H), 3.10-3.14 (m, 2H), 3.83-3.85 (m, 2H), 4.03-4.06 (m, 4H), 4.85-4.89 (m, 2H). ESI-MS C₅₇H₁₁₀NO₉⁺ [M+H]⁺ calcd. 952.8, found 952.8.

### Example 21 Synthesis of amino lipid E12LA6B6O3A2

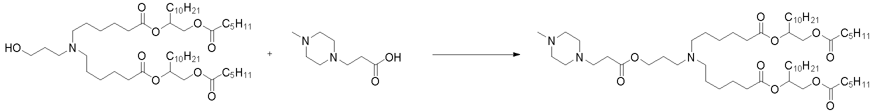

### Step 1: Synthesis of compound E12LA6B6O3

According to the method of Example 2, **E12LA6B6O3** was synthesized.

### Step 2: Synthesis of compound E12LA6B6O3A2

A solution of **E12LA6B6O3** (0.87 g, 1 mmol, 1.0 eq), N,N'-dicyclohexylcarbodiimide (0.41 g, 1 mmol, 2.0 eq), DMAP (0.12 g, 0.1 mmol, 0.1 eq), 3-(4-phenyl-piperazin-1-yl)-propionic acid (0.26 g, 1.5 mmol, 1.5 eq) in dichloromethanewas stirred at room temperature for 4 hours. TLC showed that the reaction was completed. The reaction solution was filtered, and then the filtrate was concentrated and purified using a flash column chromatography system (dichloromethane:methanol = 100:1 to 10:1) to obtain a compound **E12LA6B6O3A2** (0.80 g, 78%). ¹H NMR (400 MHz, CDCl₃) : δ 0.85-0.90 (m, 12H), 1.25-1.32 (m, 48H), 1.36-1.49 (m, 4H),1.53-1.66 (m, 12H), 2.27-2.50 (m, 25H), 2.67-2.71 (m, 2H), 4.00-4.04 (m, 2H), 4.08-4.11 (m, 2H), 4.19-4.23 (m, 2H), 5.04-5.09 (m, 2H). ESI-MS C₅₉H₁₁₂N₃O₁₀⁺ [M+H]⁺ calcd. 1022.8, found 1022.9.

### Example 22 Synthesis of mino lipid E12LA6B6O3A3

The amino lipid **E12LA6B6O3A3** can be prepared according to the same method as example 21 except replacing the 3-(4-phenyl-piperazin-1-yl)-propionic acid in Example 21 with 4-(4-methyl-1-piperazine)butyric acid, and its structural formula is as follows:

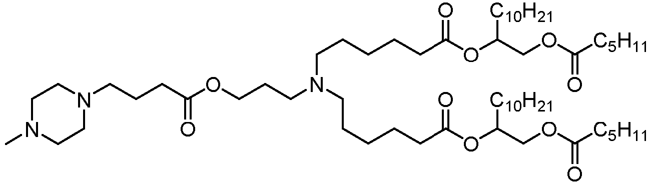

¹H NMR (400 MHz, CDCl₃) : δ 0.85-0.90 (m, 12H), 1.25-1.45 (m, 50H), 1.55-1.66 (m, 12H), 1.69-1.83 (m, 4H), 2.27-2.47 (m, 27H), 4.00-4.04 (m, 2H), 4.07-4.10 (m, 2H), 4.19-4.23 (m, 2H), 5.04-5.10 (m, 2H). ESI-MS C₆₀H₁₁₄N₃O₁₀⁺ [M+H]⁺ calcd. 1036.8, found 1036.8.

### Example 23 Synthesis of amino lipid E12LA6B6O3A4

The amino lipid **E12LA6B6O3A4** can be prepared according to the same method as example 21 except replacing the 3-(4-phenyl-piperazin-1-yl)-propionic acid in Example 21 with 3-(dimethylamino)propionic acid, and its structural formula is as follows:

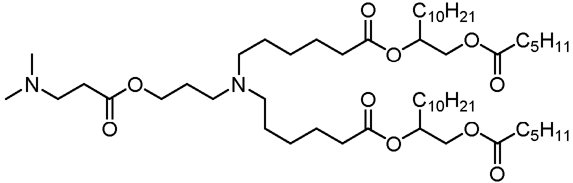

¹H NMR (400 MHz, CDCl₃) : δ 0.84-0.89 (m, 12H), 1.24-1.35 (m, 50H), 1.41-1.48 (m, 6H), 1.52-1.65 (m, 12H), 2.23 (s, 6H), 2.26-2.30 (m, 8H), 2.35-2.39 (m, 4H), 2.44-2.48 (m, 4H), 2.58-2.62 (m, 2H), 3.99-4.03 (m, 2H), 4.08-4.11 (m, 2H), 4.18-4.22 (m, 2H), 5.03-5.09 (m, 2H). ESI-MS C₅₆H₁₀₇N₂O₁₀⁺ [M+H]⁺ calcd. 967.8, found 967.8.

### Example 24 Synthesis of amino lipid E12LA6B6O3A5

The amino lipid **E12LA6B6O3A5** can be prepared according to the same method as example 21 except replacing the 3-(4-phenyl-piperazin-1-yl)-propionic acid in Example 21 with 5-(dimethylamino)pentanoic acid, and its structural formula is as follows:

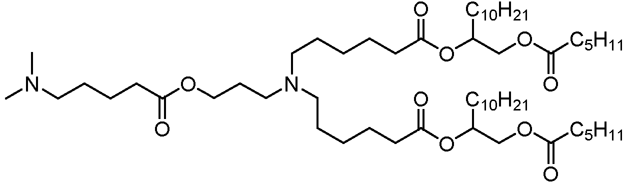

¹H NMR (400 MHz, CDCl₃) : δ 0.84-0.89 (m, 12H), 1.24-1.35 (m, 48H), 1.41-1.75 (m, 18H), 2.21 (s, 6H), 2.22-2.30 (m, 12H), 2.35-2.37 (m, 4H), 2.42-2.45 (m, 2H), 3.98-4.03 (m, 2H), 4.05-4.09 (m, 2H), 4.18-4.22 (m, 2H), 5.03-5.09 (m, 2H). ESI-MS C₅₈H₁₁₁N₂O₁₀⁺ [M+H]⁺ calcd. 995.8, found 995.9.

### Example 25 Synthesis of amino lipid E12LA6B6O3A6

The amino lipid **E12LA6B6O3A3** can be prepared according to the same method as example 21 except replacing the 3-(4-phenyl-piperazin-1-yl)-propionic acid in Example 21 with 7-(dimethylamino)heptanoic acid, and its structural formula is as follows:

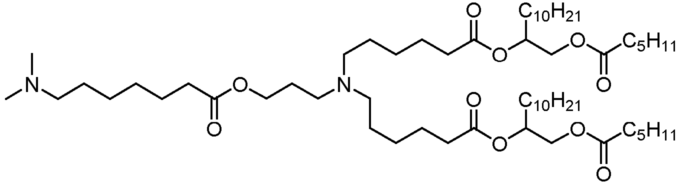

¹H NMR (400 MHz, CDCl₃) : δ 0.84-0.91 (m, 12H), 1.23-1.37 (m, 50H), 1.39-1.49 (m, 6H), 1.52-1.64 (m, 14H), 2.21 (s, 6H), 2.25-2.30 (m, 12H), 2.33-2.37 (m, 4H), 2.42-2.45 (m, 2H), 3.98-4.03 (m, 2H), 4.05-4.08 (m, 2H), 4.18-4.22 (m, 2H), 5.03-5.08 (m, 2H). ESI-MS C₆₀H₁₁₅N₂O₁₀⁺ [M+H]⁺ calcd. 1023.8, found 1023.8.

### Example 26 Synthesis of amino lipid E8LA6B6O3A6

The amino lipid **E8LA6B6O3A6** can be prepared according to the same method as example 21 except replacing **E12LA6B6O3** in Example 21 with **E8LA6B6O3,** and its structural formula is as follows:

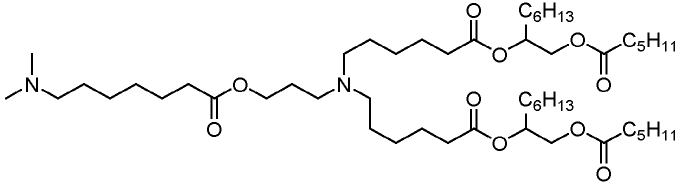

¹H NMR (400 MHz, CDCl₃) : *δ* 0.86-0.91 (m, 12H), 1.26-1.43 (m, 36H), 1.39-1.49 (m, 6H), 1.56-1.79 (m, 12H), 2.23-2.48 (m, 24H), 4.00-4.11 (m, 4H), 4.20-4.24 (m, 2H), 5.07-5.11 (m, 2H). ESI-MS C₅₂H₉₉N₂O₁₀⁺ [M+H]⁺ calcd. 911.7, found 911.9.

### Example 27 Synthesis of amino lipid E12LA6B6O3A7

The amino lipid **E12LA6B6O3A7** can be prepared according to the same method as example 21 except replacing 3-(4-phenyl-piperazin-1-yl)-propionic acid in Example 21 with 1-methylpiperidine-4-carboxylic acid, and its structural formula is as follows:

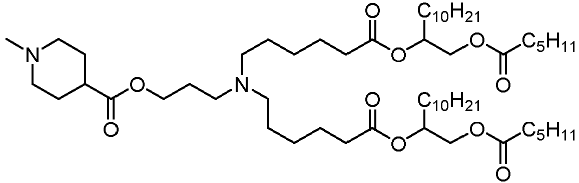

¹H NMR (400 MHz, CDCl₃) : *δ* 0.87-0.92 (m, 12H), 1.26-1.41 (m, 48H), 1.57-1.83 (m, 20H), 2.24-2.40 (m, 16H), 2.45-2.48 (m, 2H), 2.80-2.84 (m, 2H), 4.01-4.06 (m, 2H), 4.10-4.13 (m, 2H), 4.21-4.25 (m, 2H), 5.06-5.11 (m, 2H). ESI-MS C₅₈H₁₀₉N₂O₁₀⁺ [M+H]⁺ calcd. 993.8, found 993.7.

### Example 28 Synthesis of amino lipid E12LA6B6O3A8

The amino lipid **E12LA6B6O3A8** can be prepared according to the same method as example 21 except replacing 3-(4-phenyl-piperazin-1-yl)-propionic acid in Example 21 with 1-piperidine propionic acid, and its structural formula is as follows:

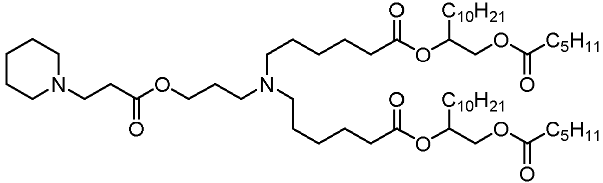

¹H NMR (400 MHz, CDCl₃) : δ 0.85-0.92 (m, 12H), 1.24-1.36 (m, 44H), 1.40-1.45 (m, 6H), 1.54-1.69 (m, 12H), 2.27-2.30 (m, 8H), 2.34-2.52 (m, 12H), 2.63-2.67 (m, 2H), 4.00-4.04 (m, 2H), 4.07-4.10 (m, 8H), 4.19-4.23 (m, 2H), 5.03-5.09 (m, 2H). ESI-MS C₅₉H₁₁₁N₂O₁₀⁺ [M+H]⁺ calcd. 1007.8, found 1007.9.

### Example 29 Synthesis of amino lipid E12LA12B6O30A9

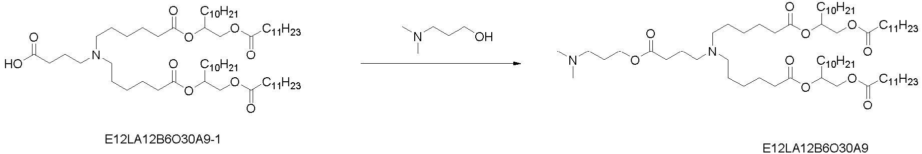

### Step 1: Synthesis of compound E12LA12B6O30A9-1

**According to the method of Example 16, E12LA12B6O30A9-1** was synthesized.

### Step 2: Synthesis of compound E12LA12B6O3A9

A solution **of E12LA12B6O30A9-1** (1.06 g, 1 mmol, 1.0 eq), N,N'-dicyclohexylcarbodiimide (0.41 g, 2 mmol, 2.0 eq), DMAP (0.12 g, 0.1 mmol, 0.1 eq), and 3- dimethylaminopropanol (0.16 g, 1.5 mmol, 1.5 eq) in dichloromethane was stirred at room temperature for 4 hours. TLC showed that the reaction was completed. The reaction solution was filtered, and then the filtrate was concentrated and purified using a flash column chromatography system (dichloromethane:methanol =50:1 to 10:1 ) to obtain a compound **E12LA12B6O3A9-1** (0.92 g, 80%), ¹H NMR (400 MHz, CDCl₃) : δ 0.86-0.91 (m, 12H), 1.24-1.42 (m, 68H), 1.45-1.72 (m, 20H), 1.84-1.86 (m, 2H), 2.26 (s, 6H), 2.30-2.42 (m, 12H), 2.60-2.63 (m, 2H), 3.78-3.81, (m, 2H), 4.01-4.15 (m, 4H), 4.22-4.26 (m, 2H), 5.06-5.12 (m, 2H). ESI-MS C₆₉H₁₃₃N₂O₁₀⁺ [M+H]⁺ calcd. 1050.0, found 1050.3.

### Example 30 Synthesis of amino lipid E12LA12B6O30A10

The amino lipid E12LA12B6030A10 can be prepared according to the same method as example 29 except replacing 3-dimethylaminopropanol in Example 29 with 3-dimethylaminopropylamine, and its structural formula is as follows:

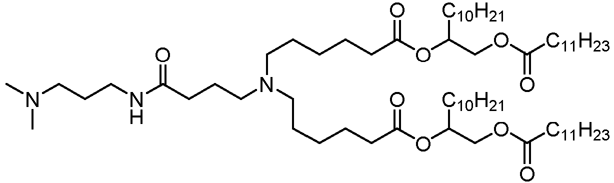

¹H NMR (400 MHz, CDCl₃) : δ 0.86-0.91 (m, 12H), 1.24-1.42 (m, 68H), 1.45-1.84 (m, 22H), 2.28 (s, 6H), 2.32-2.44 (m, 12H), 2.60-2.63 (m, 2H), 3.50-3.54, (m, 2H), 3.78-3.82 (m, 2H), 4.00-4.04 (m, 2H), 4.22-4.26 (m, 2H), 5.06-5.12 (m, 2H). ESI-MS C₆₉H₁₃₄N₃O₉⁺ [M+H]⁺ calcd. 1149.0, found 1149.0.

### Example 31 Synthesis of amino lipid E12LA12B6O3A11

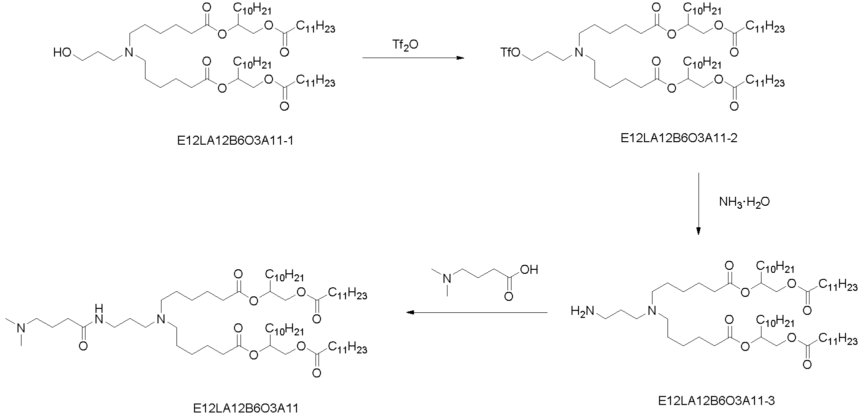

### Step 1: Synthesis of compound E12LA12B6O3A11-1

According to the method of Example 9, **E12LA6B6O3A11-1** was synthesized.

### Step 2: Synthesis of compound E12LA6B6O3A11-1-2

A 50 mL round-bottom flask was taken and filled with nitrogen by evacuation. The compound E12LA6B603A11-1(5.18 g, 5 mmol, 1.0 eq) and anhydrous dichloromethane (20 mL) were added into the flask and the mixture was stirred at 0°C for 10 minutes. Trifluoromethanesulfonic anhydride (2.12 g, 7.5 mmol, 1.5 eq) was added dropwise to the mixture and the resultant was stirred at 0°C for 10 minutes. 2,6-Dimethylpyridine (0.80 g, 7.5 mmol, 1.5 eq) was added and the reaction solution was stirred at room temperature overnight. The reaction solution was washed once with water and a saturated sodium chloride solution respectively, dried over anhydrous sodium sulfate, concentrated, and purified using a flash column chromatography system(dichloromethane:methanol=50:1 to 20:1) to obtain a compound E12LA6B603A11-2(4.38 g, 75%), ESI-MS C₆₄H₁₂₁F₃NO₁₁S⁺ [M+H]⁺ calcd. 1168.9, found 1169.0.

### Step 3: Synthesis of compound E12LA6B6O3A11-3

E12LA6B603A11-2 (4.38 g, 3.75 mmol, 1.0 eq), 20 mL tetrahydrofuran, and 5 mL ammonia water (25% by mass aqueous solution) were added into a 100 mL pressure bottle, and the mixture was stirred at 100 °C overnight. The reaction solution is washed once with water and a saturated sodium chloride solution respectively, dried over anhydrous sodium sulfate, concentrated, and purified using a flash column chromatography system (dichloromethane : methanol=20:1 to 5:1) to obtain a compound E12LA6B603A11-3(1.71 g, 44%), ESI-MS C₆₃H₁₂₃N₂O₈⁺ [M+H]⁺ calcd. 1035.9, found 1035.9.

### Step 4: Synthesis of compound E12LA6B6O3A11

A solution of E12LA6B603A11-3 (1.71 g, 1.65 mmol, 1.0 eq), N,N'-dicyclohexylcarbodiimide (0.68 g, 3.3 mmol, 2.0 eq), DMAP (0.20 g, 0.16 mmol, 0.1 eq), 4-dimethylaminobutyric acid (0.32 g, 2.48 mmol, 1.5 eq) in dichloromethane was stirred at room temperature for 4 hours. TLC showed that the reaction was completed. The reaction solution was filtered, and then the filtrate was concentrated and purified using a flash column chromatography system (dichloromethane:methanol =50:1 to 10:1) to obtain a compound **E12LA6B6O3A11** (1.48 g, 78%), ¹H NMR (400 MHz, CDCl₃) : δ 0.85-0.91 (m, 12H), 1.24-1.41 (m, 68H), 1.45-1.82 (m, 22H), 2.27 (s, 6H), 2.32-2.42 (m, 12H), 2.61-2.63 (m, 2H), 3.36-3.42, (m, 2H), 3.77-3.79 (m, 2H), 4.01-4.04 (m, 2H), 4.23-4.26 (m, 2H), 5.04-5.10 (m, 2H). ESI-MS C₆₉H₁₃₄N₃O₉⁺ [M+H]⁺ calcd. 1049.0, found 1049.1.

### Example 32 Synthesis of amino lipid E12N1LA6B6O3

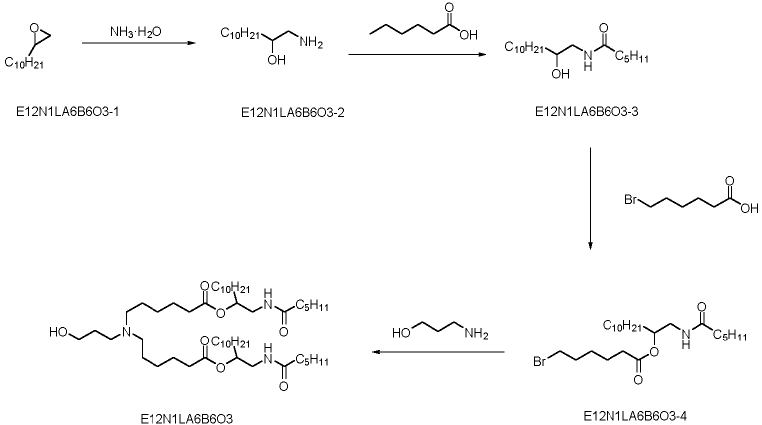

### Step 1: Synthesis of compound E12NILA6B6O3-2

A mixture of 1,2-epoxydodecane (3.69 g, 20 mmol, 1.0 eq), 20 mL of aqueous ammonia (25% by mass aqueous solution), 10 mL of ethanol, and 10 mL of water was stirred at 60°C. for 16 hours. The reaction solution was spin-dried, 3 mL of ethanol and 30 mL of n-heptane were added and slurried at 60°C for 4 hours. The resultant was filtered, and the filter cake was dried at 50°C for 10 hours to obtain a compound E12N1LA6B6O3-2(3.46 g, 86%), ESI-MS C₁₂H₂₈NO⁺ [M+H]⁺ calcd. 202.2, found 202.2.

### Step 2: Synthesis of compound E12N1LA6B6O3-3

A solution of compound **E12N1LA6B6O3-2(3.46** g, 17.2 mmol, 1.5 eq), hexanoic acid (1.34 g, 11.5 mmol, 1.0 eq), 1-(3-dimethylaminopropyl)-3-ethylcarbodiimide hydrochloride (3.30 g, 17.2 mmol, 1.5 eq), N,N-diisopropylethylamine (2.22 g, 17.2 mmol, 1.5 eq), and 4-dimethylaminopyridine (2.10 g, 17.2 mmol, 1.5 eq) in dichloromethane (40 mL) was stirred at room temperature overnight. The reaction solution was extracted three times with dichloromethane, washed once with saturated brine, dried over anhydrous sodium sulfate, concentrated, and purified using a flash column chromatography system (n-heptane:ethyl acetate =10:1 to 3:1 ) to obtain a compound **E12N1LA6B6O3-3(1.10** g, 32%), ESI-MS C₁₈H₃₈NO₂⁺ [M+H]⁺ calcd. 300.3, found 300.2.

### Step 3: Synthesis of compound E12N1LA6B6O3-4

A solution of compound **E12N1LA6B6O3-3(1.10** g, 3.68 mmol, 1.0 eq), hexanoic acid(0.51 g, 4.42 mmol, 1.2 eq), 1-(3-dimethylaminopropyl)-3-ethylcarbodiimide hydrochloride(1.41 g, 7.36 mmol, 2.0 eq), N,N-diisopropylethylamine(1.90 g, 14.2 mmol, 4.0 eq), and 4-dimethylaminopyridine(0.04 g, 0.37 mmol, 0.1 eq) in dichloromethane (20 mL) was stirred at room temperature overnight. TLC showed that the reaction was completed. The reaction solution was extracted three times with dichloromethane, washed once with saturated brine, dried over anhydrous sodium sulfate, concentrated, and purified using a flash column chromatography system (n-heptane:ethyl acetate =100:1 to 20:1) to obtain a compound **E12N1LA6B6O3-4(1.42** g, 81%), ESI-MS C₂₄H₄₇BrNO₃⁺ [M+H]⁺ calcd. 476.3, found 476.3.

### Step 4: Synthesis of compound E12N1LA6B6O3

A solution of compound **E12N1LA6B6O3-4(1.42** g, 2.99 mmol, 3.0 eq), 3-aminopropanol (0.075 g, 1.00 mmol, 1.0 eq), potassium carbonate (0.28 g, 2.00 mmol, 2.0 eq), and sodium iodide (0.15 g, 1.00 mmol, 1.0 eq) in acetonitrile (10 mL) was stirred at 75°C overnight. After the reaction solution was concentrated, it was purified using a flash column chromatography system(dichloromethane:methanol =100:1 to 20:1) to obtain a compound E12N1LA6B6O3 (0.68g, 78%). ¹H NMR (400 MHz, CDCl₃) : δ 0.86-0.93 (m, 12H), 1.25-1.37 (m, 48H), 1.41-1.69 (m, 16H), 2.14-2.18 (m, 8H), 2.30-2.43 (m, 6H), 3.34-3.48 (m, 4H), 4.90-4.96 (m, 2H). ESI-MS C₅₁H₁₀₀N₃O₇⁺ [M+H]⁺ calcd. 866.8, found 866.7.

### Example 33 Synthesis of amino lipid E12N1LA8B6O3

The amino lipid **E12N1LA8B6O3** can be prepared according to the same method as example 32 except replacing hexanoic acid in Example 32 with caprylic acid, and its structural formula is as follows:

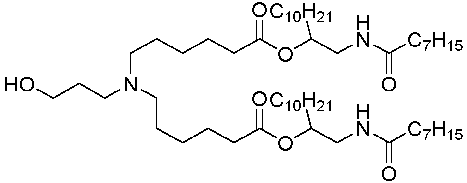

¹H NMR (400 MHz, CDCl₃) : δ 0.86-0.93 (m, 12H), 1.25-1.37 (m, 48H), 1.42-1.70 (m, 18H), 2.14-2.18 (m, 8H), 2.31-2.43 (m, 6H), 3.35-3.48 (m, 4H), 4.90-4.96 (m, 2H). ESI-MS C₅₅H₁₀₈N₃O₇⁺ [M+H]⁺ calcd. 922.8, found 923.0.

### Example 34 Synthesis of amino lipid E12N2LA6B6O3

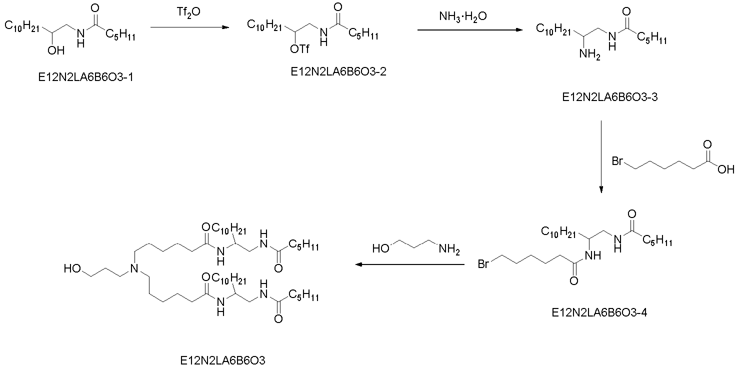

### Step 1: Synthesis of compound E12N2LA6B6O3-1

According to steps 1 and 2 of Example 32, E12N2LA6B6O3-1 was synthesized.

### Step 2: Synthesis of compound E12N2LA6B6O3-2

A 50 mL round-bottom flask was taken and filled with nitrogen by evacuation. The compound **E12N2LA6B6O3-1-1(1.50** g, 5 mmol, 1.0 eq) and anhydrous dichloromethane (20mL) were added into flask. The mixture was stirred at 0°C for 10 minutes. Trifluoromethanesulfonic anhydride (2.12 g, 7.5 mmol, 1.5 eq) was added dropwise to the mixture and the resultant was stirred at 0°C for 10 minutes. 2,6-Dimethylpyridine (0.80g, 7.5 mmol, 1.5eq) was added and the reaction solution was stirred at room temperature overnight. The reaction solution was washed once with water and saturated sodium chloride solution respectively, dried over anhydrous sodium sulfate, concentrated, and purified using a flash column chromatography system (dichloromethane:methanol=50:1to 20:1) to obtain a compound E12N2LA6B6O3-2(1.51 g, 70%), ESI-MS C₁₉H₃₇F₃NO₄S⁺ [M+H]⁺ calcd. 432.2, found 432.2.

### Step 3: Synthesis of compound E12N2LA6B6O3-3

E12N2LA6B6O3-2 (1.51 g, 3.50 mmol, 1.0 eq), 20 mL tetrahydrofuran, and 5 mL ammonia water (25% by mass aqueous solution) were added into a 100 mL pressure bottle, and the mixture was stirred at 100°C overnight. The reaction solution was washed once with water and a saturated sodium chloride solution respectively, dried over anhydrous sodium sulfate, concentrated, and purified using a flash column chromatography system (dichloromethane:methanol=20:1 to 5:1) to obtain a compound **E12N2LA6B6O3-3(0.47** g, 45%), ESI-MS C₁₈H₃₉N₂O⁺ [M+H]⁺ calcd. 299.3, found 299.3.

### Step 4: Synthesis of compound E12N2LA6B6O3-4

A solution of compound **E12N2LA6B6O3-3** (0.74 g, 1.58 mmol, 3.0 eq), N,N'-dicyclohexylcarbodiimide (0.65 g, 3.15 mmol, 2.0 eq), DMAP (0.20 g, 0.16 mmol, 0.1 eq), N,N-diisopropylethylamine(0.82 g, 6.32 mmol, 4.0 eq), and 6-bromohexanoic acid (0.46 g, 2.37 mmol, 1.5 eq) in dichloromethanewas stirred at room temperature for 4 hours. TLC showed that the reaction was completed. The reaction solution was filtered, and then the filtrate was concentrated and purified using a flash column chromatography system(n-heptane:ethyl acetate =50:1 to 1:1) to obtain a compound **E12N2LA6B6O3-4** (1.48 g, 88%), ESI-MS C₂₄H₄₈BrN₂0O₂⁺ [M+H]⁺ calcd. 475.3, found 475.3.

### Step 5: Synthesis of compound E12N2LA6B6O3

A solution of compound **E12N2LA6B6O3-4** (1.48 g, 1.39 mmol, 3.0 eq), 3-aminopropanol (0.035 g, 0.46 mmol, 1.0 eq), potassium carbonate (0.13 g, 0.92 mmol, 2.0 eq), sodium iodide(0.069 g, 0.46 mmol, 1.0 eq) in acetonitrile (10 mL) was stirred at 75°C overnight. After the reaction solution was concentrated, it was purified using a flash column chromatography system (dichloromethane : methanol =100:1 to 20:1) to obtain a compound **E12N2LA6B6O3(0.97** g, 81%). ¹H NMR (400 MHz, CDCl₃) : δ 0.86-0.92 (m, 12H), 1.25-1.37 (m, 42H), 1.41-1.69 (m, 18H), 2.06-2.18 (m, 12H), 2.30-2.42 (m, 4H), 3.32-3.48 (m, 4H), 3.70-3.74 (m, 2H), 4.90-4.96 (m, 2H). ESI-MS C₅₁H₁₀₂N₅O₅⁺ [M+H]⁺ calcd. 864.8, found 864.7.

### Example 35 Synthesis of amino lipid E12S1LA6B6O3

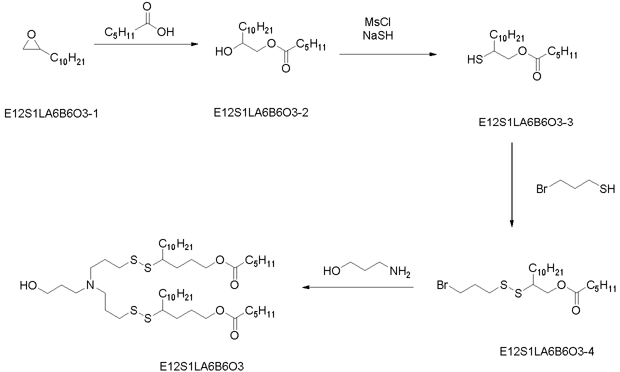

### Step 1: Synthesis of compound E12S1LA6B6O3-2

Ferric chloride (16.2 mg, 0.1 mmol, 0.025 eq), pyridine (4 mg, 0.05 mmol, 0.0125 eq), hexanoic acid (464 mg, 4.0 mmol, 1.0 eq) and 1,2-epoxydodecane (884 mg, 4.8 mmol, 1.2 eq) were added in sequence into a 25 mL reaction tube. The reaction solution was stirred at room temperature overnight. After the reaction was completed, the reaction solution was concentrated and purified using a flash column chromatography system (n-heptane :ethyl acetate=50:1 to 10:1) to obtain a compound **E12S1LA6B6O3-2(1.2** g, 90.0%). ESI-MS C₁₈H₃₇O₃⁺ [M+H]⁺ calcd. 301.3, found 301.3.

### Step 2: Synthesis of compound E12S1LA6B6O3-3

**E12S1LA6B6O3-2(1.2** g, 3.6 mmol, 1.0 eq), triethylamine (0.36 g, 3.6 mmol, 1.0 eq), methanesulfonyl chloride (0.49 g, 4.32 mmol, 1.2 eq), and 10mL of dichloromethanewere added into a 25 mL reaction tube. The mixture was stirred at room temperature for 2 hours. The reaction solution was washed once with water and a saturated sodium chloride solution respectively, dried over anhydrous sodium sulfate, filtered, and the filtrate was concentrated. 10 mL of N,N-dimethylformamide and sodium hydrosulfide (0.24 g, 4.32 mmol, 1.2 eq) were added, and the mixture was stirred at 45°C overnight. The reaction solution was extracted three times with ethyl acetate, washed once with saturated brine, dried over anhydrous sodium sulfate, concentrated, and purified using a flash column chromatography system (n-heptane : ethyl acetate =100:1 to 10:1) to obtain a compound **E12S1LA6B6O3-3(0.69g,** 61%), ESI-MS C₁₈H₃₇O₂S⁺ [M+H]⁺ calcd. 317.3, found 317.2.

### Step 3: Synthesis of compound E12S1LA6B6O3-4

**E12S1LA6B6O3-3(0.69** g, 2.2 mmol, 1.0 eq), dibromohydantoin (0.13 g, 0.44 mmol, 0.2 eq), 3-bromopropane-1-thiol (0.41 g, 2.64 mmol, 1.2 eq), and 10 mL of dichloromethane were added into a 25 mL reaction tube. The mixture was stirred at room temperature for 1 hour. The reaction solution was extracted three times with ethyl acetate, washed once with saturated brine, dried over anhydrous sodium sulfate, concentrated, and purified using a flash column chromatography system (n-heptane:ethyl acetate =100:1 to 10:1) to obtain a compound **E12S1LA6B6O3-4(0.70g,** 68%), ESI-MS C₂₁H₄₂BrO₂S₂⁺ [M+H]⁺ calcd. 469.2, found 469.2.

### Step 4: Synthesis of compound E12S1LA6B6O3

A solution of compound **E12S1LA6B6O3-4** (0.70 g, 1.50 mmol, 3.0 eq), 3-aminopropanol (0.038 g, 0.50 mmol, 1.0 eq), potassium carbonate (0.14 g, 1.00 mmol, 2.0 eq), and sodium iodide (0.075 g, 0.50 mmol, 1.0 eq) in acetonitrile (10 mL) was stirred at 75°C overnight. After the reaction solution was concentrated, it was purified using a flash column chromatography system (dichloromethane : methanol =100:1 to 20:1) to obtain a compound **E12S1LA6B6O3(1.09g,** 80%). ¹H NMR (400 MHz, CDCl₃) : δ 0.86-0.91 (m, 12H), 1.25-1.35 (m, 40H), 1.41-1.54 (m, 12H), 1.59-1.67 (m, 10H), 2.09-2.18 (m, 10H), 2.34-2.40 (m, 4H), 2.50-2.54 (m, 2H), 3.64-3.72 (m, 2H), 4.00-4.06 (m, 4H). ESI-MS C₄₉H₉₈NO₅S₄⁺ [M+H]⁺ calcd. 908.6, found 908.7.

### Preparation of lipid nanoparticles

### Example 36 Preparation of lipid nanoparticles

The amino lipid compound of the present invention (or DLin-MC3 or SM-102 (both purchased from AVT (Shanghai) Pharmaceutical Technology Co., Ltd.)), phospholipid lipid (DSPC), cholesterol, and PEGylated lipid (DMG-PEG2000) were mixed and dissolved in anhydrous ethanol at a molar ratio of 47.5:10:41:1.5. The resulting ethanol solution and citric acid buffer (50 mM, pH = 4.0) dissolved with Luc-mRNA (TriLink) were mixed in a microfluidic chip at a volume ratio of 1:3 using a microfluidic preparation system to prepare a crude solution of lipid nanoparticles, which was then dialyzed in 1X PBS at 4°C for 6 h using a dialysis cassette (Fisher, MWCO 20,000). The product was filtered through a 0.22 µm microporous filter membrane before use. The mass ratio of the amino lipid compound to luciferase mRNA (Luc mRNA) was about 40:1.

The obtained lipid nanoparticles were characterized and the results were shown in Table 1.

The particle size and PDI (polydispersity index) of the prepared lipid nanoparticles were measured by Nano-ZSZEN3600 (Malvern). 20 µL of lipid nanoparticle (LNP) solution was taken for particle size measurement, and cycled three times with each cycle for 30 s.

The encapsulation efficiency was determined according to the standard procedure of the Quant-iT RiboGreen RNA Kit.

**Table 1 Characterization data of LNPs prepared using representative amino lipid compounds**

| Item | Amino lipid No. | Average particle size (nm) | PDI | Encapsulation efficiency (%) |
|---|---|---|---|---|
| LNP-1 | E8LA8B6O3 | 84 | 0.07 | 90.03 |
| LNP-2 | E8LA10B6O3 | 73 | 0.09 | 93.95 |
| LNP-3 | E8LA12B6O3 | 72 | 0.09 | 95.67 |
| LNP-4 | E10LA6B6O3 | 92 | 0.07 | 95.41 |
| LNP-5 | E10LA8B6O3 | 71 | 0.11 | 92.23 |
| LNP-6 | E10LA10B6O3 | 70 | 0.06 | 95.90 |
| LNP-7 | E12LA6B6O3 | 75 | 0.08 | 98.26 |
| LNP-8 | E12LA8B6O3 | 73 | 0.10 | 94.90 |
| LNP-9 | E12LA12B6O3 | 73 | 0.09 | 97.5% |
| LNP-10 | E8CA5B6O3 | 69 | 0.06 | 95.8% |
| LNP-12 | E12LA6B6O9 | 95 | 0.11 | 93.2% |
| LNP-13 | E12LA6B6O10 | 102 | 0.10 | 93.0% |
| LNP-14 | E12LA6B6O12 | 97 | 0.05 | 97.6% |
| LNP-15 | E12LA6B6O13 | 83 | 0.01 | 94.4% |
| LNP-16 | E12LA6B6O15 | 90 | 0.12 | 95.2% |
| LNP-17 | E12LA12B6O30 | 127 | 0.03 | 98.2% |
| LNP-18 | E12LA12B6O31 | 130 | 0.04 | 98.4% |
| LNP-19 | K3LA6B6O3 | 129 | 0.08 | 98.8% |
| LNP-20 | K4LA6B6O3 | 120 | 0.06 | 98.9% |
| LNP-21 | K5LA6B6O3 | 106 | 0.03 | 96.2% |
| LNP-23 | E12LA6B6O3A3 | 53 | 0.07 | 94.6% |
| LNP-24 | E12LA6B6O3A4 | 80 | 0.03 | 90.7% |
| LNP-25 | E12LA6B6O3A5 | 117 | 0.08 | 97.8% |
| LNP-26 | E12LA6B6O3A6 | 103 | 0.07 | 98.5% |
| LNP-27 | E8LA6B6O3A6 | 108 | 0.07 | 97.6% |
| LNP-28 | E12LA6B6O3A7 | 110 | 0.05 | 96.5% |
| LNP-29 | E12LA6B6O3A8 | 124 | 0.06 | 95.3% |
| LNP-30 | E12LA12B6O30A9 | 121 | 0.14 | 92.4% |
| LNP-31 | E12LA12B6O30A10 | 117 | 0.08 | 91.8% |
| LNP-32 | E12LA12B6O3A11 | 132 | 0.09 | 95.6% |
| LNP-33 | E12N2LA6B6O3 | 168 | 0.06 | 90.3% |
| Comparative LNP-1 | DLin-MC3 | 72 | 0.12 | 89.74 |
| Comparative LNP-2 | SM-102 | 79 | 0.10 | 94.32 |

From the above results, it can be seen that the lipid nanoparticles provided by the present invention all have an encapsulation efficiency higher than that of DLin-MC3, and most of the lipid nanoparticles have an encapsulation efficiency higher than that of SM-102.

### Example 37 Transfection of lipid nanoparticles prepared from amino lipid compounds on primary BMDC cells

Animal preparation: 6-week-old female C57BL/6 mice weighing about 20 g were selected and raised in an SPF-grade feeding room. Animal experiments were conducted strictly in accordance with the guidelines of the National Institutes of Health and animal ethics requirements.

Cell acquisition: C57BL/6 mice were sacrificed by cervical dislocation and immersed in 75% alcohol for 5 minutes for disinfection. The thigh and tibia of the mice were dissected and the attached muscles were removed to expose the bone. The bone marrow in the tibia was blown out with a 1 mL syringe filled with PBS. The obtained bone marrow was dispersed by blowing and filtered through a 50 µm filter mesh to remove impurities. Red blood cell lysis solution (3-4 mL) was added to the obtained filtrate and allowed to stand for 5 minutes. Then, the supernatant was removed by centrifugation at 800 g for 5 minutes. The obtained cells were placed in 1640 culture medium (containing 10% fetal bovine serum, 20 ng/mL GMCSF, 10 ng/mL IL4) for being resuspended, and inoculated into 6-well plates at a density of 100,000 cells/mL of culture medium. The plates were placed in a cell culture incubator containing 5% CO₂ at 37°C, and the medium was half-changed every 2 days. On the seventh day, the suspended cells and loosely adherent cells were collected and inoculated into 96-well all-white ELISA plates at a density of 20,000 cells per well, with a culture medium volume of 100 µL.

Cell transfection: the lipid nanoparticles encapsulating luciferase mRNA were added to a 96-well all-white ELISA plate with spreaded primary cells, and the volume of mRNA lipid nanoparticles added to each well was controlled to be 10 µL. Then, the plate was placed in an incubator with 5% CO₂ concentration at 37°C for 12 hours.

Transfection efficiency detection: 20 µL of substrate ONE-Glo^{™} Luciferase was added to each well of a 96-well all-white ELISA plate, and the assay was performed using a multifunctional microplate reader (Biorek SynergyH1) after 1 min. The expression intensity of Luc mRNA transfected on BMDCs by lipid nanoparticles (LNPs) composed of representative amino lipid compounds is shown in Table 2, with DLin-MC3 and SM0102 as controls.

**Table 2 Expression intensity of transfection of representative amino lipid compounds on BMDCs**

| Item | Amino lipid No. | Fluorescence intensity |
|---|---|---|
| LNP-1 | E8LA8B6O3 | 1.3E+05 |
| LNP-2 | E8LA10B6O3 | 5.2E+04 |
| LNP-4 | E10LA6B6O3 | 1.5E+05 |
| LNP-6 | E10LA10B6O3 | 6.9E+04 |
| LNP-7 | E12LA6B6O3 | 1.8E+05 |
| LNP-8 | E12LA8B6O3 | 7.6E+04 |
| LNP-9 | E12LA12B6O3 | 4.1E+05 |
| LNP-10 | E8CA5B6O3 | 2.3E+05 |
| LNP-11 | E12LA6B6O3 | 5.4E+05 |
| LNP-12 | E12LA6B6O9 | 2.2 E+05 |
| LNP-14 | E12LA6B6O12 | 4.9 E+04 |
| LNP-18 | E12LA12B6O31 | 9.6 E+04 |
| LNP-19 | K3LA6B6O3 | 5.4E+05 |
| LNP-20 | K4LA6B6O3 | 4.9 E+05 |
| LNP-21 | K5LA6B6O3 | 4.8E+05 |
| LNP-22 | E12LA6B6O3A2 | 4.0E+05 |
| LNP-25 | E12LA6B6O3A5 | 1.5 E+05 |
| LNP-27 | E8LA6B6O3A6 | 2.8E+05 |
| LNP-28 | E12LA6B6O3A7 | 7.3 E+04 |
| LNP-29 | E12LA6B6O3A8 | 1.9 E+05 |
| LNP-30 | E12LA12B6O30A9 | 2.7 E+05 |
| LNP-31 | E12LA12B6O30A10 | 4.1E+05 |
| LNP-32 | E12LA12B6O3A11 | 1.4E+05 |
| Comparative LNP-1 | DLin-MC3 | 4.1E+04 |
| Comparative LNP-2 | SM-102 | 1.0E+05 |

It can be seen that the expression intensities of the amino lipids provided by the present invention are significantly better than that of DLin-MC3, and the expression intensities of some of the amino lipids are better than that of SM-102.

### Example 38 Evaluation of the in vivo delivery performance of lipid nanoparticles for luciferase mRNA

Preparation of lipid nanoparticles: same as Example 36.

### Animal experiments

Animal preparation: 6-week-old female C57BL/6 mice weighing about 20 g were selected and raised in an SPF-grade feeding room. Animal experiments were conducted strictly in accordance with the guidelines of the National Institutes of Health and animal ethics requirements.

In vivo delivery: Five C57BL/6 mice were randomly selected from each group and injected with lipid nanoparticle solution at a dose of 0.5 mg/kg mRNA by intramuscular injection. Twelve hours later, 200 µL of 10 mg/mL D-luciferin potassium salt was injected into the peritoneal cavity of each mouse. Five minutes later, the mice were placed under an in vivo imaging system (IVIS-200, Xenogen) to observe the total fluorescence intensity of each mouse and take photos for recording. The expression intensity of Luc mRNA delivered by intramuscular injection of LNPs composed of representative amino lipid compounds is shown in Table 3, with DLin-MC3 and SM-102 as controls.

**Table 3 Expression intensity of Luc mRNA delivered by intramuscular injection of LNPs composed of representative amino lipids**

| No. | Amino lipid No. | Fluorescence intensity |
|---|---|---|
| 1 | E8LA8B6O3 | 7.7E+08 |
| 2 | E10LA6B6O3 | 1.4E+09 |
| 3 | E10LA10B6O3 | 1.2E+09 |
| 4 | E12LA6B6O3 | 2.2E+09 |
| 5 | E12LA8B6O3 | 8.2E+08 |
| 6 | E8LA10B6O3 | 1.6E+09 |
| 7 | E12LA6B6O10 | 1.6E+09 |
| 8 | E10LA8B6O3 | 1.8E+09 |
| 9 | E12LA6B6O3A6 | 1.5E+09 |
| 10 | K3LA6B6O3 | 2.0E+09 |
| 11 | DLin-MC3 | 7.6 E+08 |
| 12 | SM-102 | 1.4E+09 |

### Example 39 Evaluation of in vivo immune and tumor therapeutic effects of lipid nanoparticles prepared from amino lipid compounds

Preparation method: The amino lipid compound of the present invention was mixed with DSPC, cholesterol and DMG-PEG2000 at a molar ratio of 47.5:10:41:1.5 and dissolved in anhydrous ethanol. The obtained ethanol solution and citric acid buffer (50 mM, pH = 4.0) dissolved with OVA-mRNA were mixed in a microfluidic chip at a volume ratio of 1:3 using a microfluidic preparation system to prepare lipid nanoparticles, and then dialyzed in 1X PBS at 4°C for 6 h using a dialysis cassette (Fisher, MWCO 20,000) and filtered with a 0.22 µm microporous filter membrane before use. The mass ratio of the amino lipid compound to ovalbumin mRNA (OVA mRNA) was about 40: 1.

Animal preparation: Female C57BL/6 mice aged 5-6 weeks, weighing about 18-20 g were selected and kept in an SPF-grade feeding room. Animal experiments were conducted strictly in accordance with the guidelines of the National Institutes of Health and animal ethics requirements.

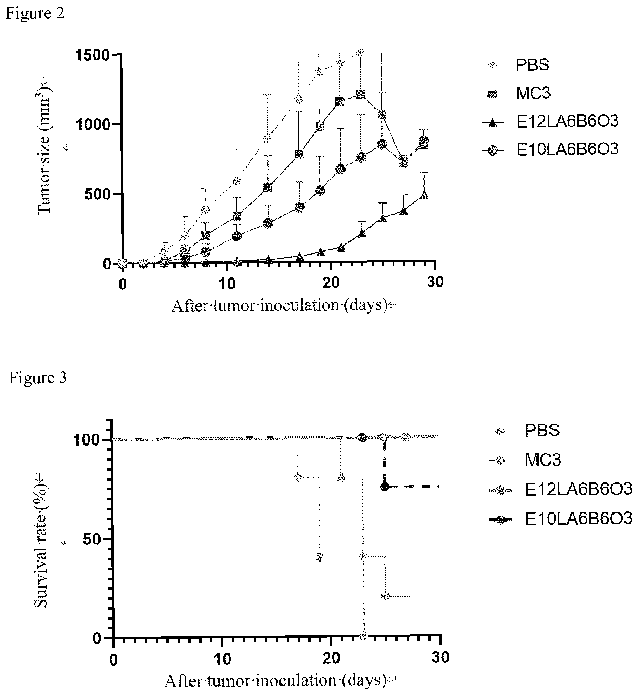

In vivo delivery: B16-OVA melanoma cells (1.5 × 10⁵) were injected subcutaneously into the lateral thigh of mice. Vaccination was started when the tumor size reached 50 mm³(approximately day 6 or 7 after tumor inoculation). Animals were immunized twice by intramuscular injection of LNP formulation containing 1 µg of OVA-mRNA, with an interval of the second injection from the first injection being 7 days. Tumor growth was measured three times per week using a digital caliper and calculated as 0.5 × length × width × width. Mice were euthanized when tumor volume reached 1500 mm³. The tumor growth rates of the **E12LA6B6O3** group and the **E10LA6B6O3** group were significantly slower than that of the MC3 group (as shown in Figure 2), and 100% (**E12LA6B6O3** group) and 80% (**E10LA6B6O3** group) of the mice achieved complete remission, which was significantly better than that of the MC3 group (as shown in Figure 3).

### Example 40 Evaluation of the in vivo delivery performance of luciferase mRNA in LNPs composed of different phospholipid lipids ratios

Preparation of lipid nanoparticles: the ionizable lipid (E12LA6B6O3) content was kept at 42.5%, the PEGylated lipid DMG-PEG2000 content was kept at 1.5%, and the phospholipid lipid ratio was adjusted to 15, 12.5, 10, 7.5, 5.0, 2.5, and 0. The increased or decreased molar ratio during adjustment of the phospholipid lipid molar ratio was applied to the cholesterol ratio to verify its effect on the quality control evaluation of lipid nanoparticles and on the in vivo delivery performance of luciferase mRNA. The groups were numbered B1-B7, and the specific proportions were shown in Table 4. ALC-0315 and SM-102 were used as positive controls.

**Table 4: Component ratios under different phospholipids**

| No. | Ionizable lipid: phospholipid lipid: cholesterol: PEGylated lipid |
|---|---|
| B1 | 40: 0: 58.5: 1.5 |
| B2 | 40: 2.5: 56: 1.5 |
| B3 | 40: 5: 53.5: 1.5 |
| B4 | 40: 7.5: 51: 1.5 |
| B5 | 40: 10: 48.5: 1.5 |
| B6 | 40: 12.5: 46: 1.5 |
| B7 | 40: 15: 43.5: 1.5 |

E12LA6B6O3, phospholipid lipid (DSPC), cholesterol, and PEGylated lipid DMG-PEG2000 were mixed and dissolved in anhydrous ethanol according to the molar ratio in Table 4. The obtained ethanol solution and citric acid buffer (50 mM, pH = 4.0) dissolved with Luc-mRNA (TriLink) were mixed in a microfluidic chip at a volume ratio of 1:3 using a microfluidic preparation system to prepare a crude solution of lipid nanoparticles, which was then dialyzed in 1X PBS at 4°C for 6 h using a dialysis cassette (Fisher, MWCO 20,000). The product was filtered through a 0.22 µm microporous filter membrane before use. The base molar ratio of the amino lipid compound to the luciferase mRNA (Luc mRNA) is about 6.5:1. The quality control of lipid nanoparticles was tested on a Malvern particle size analyzer and an microplate reader, and SM-102 and ALC-0315 (both purchased from AVT (Shanghai) Pharmaceutical Technology Co., Ltd) were added as a control group.

Animal preparation: 6-week-old female Balb/C mice weighing about 20 g were selected and raised in an SPF-grade feeding room. Animal experiments were conducted in strict accordance with the guidelines of the National Institutes of Health and animal ethics requirements.

In vivo delivery: Three Balb/C mice were randomly selected from each group and injected with lipid nanoparticle solution at a dose of 0.1 mg/kg mRNA via tail vein injection. After 6 hours, 150 µL of 15 mg/mL D-luciferin potassium salt was injected into the peritoneal cavity of each mouse. After 10 minutes, the mice were placed under the in vivo imaging system (IVIS Spectrum) to observe the total fluorescence intensity of each mouse and take photos for recording. The expression intensity of Luc mRNA delivered by tail vein injection of LNPs with different DSPC contents is shown in Table 5.

**Table 5: Quality control data and expression intensity of LNPs with different phospholipid lipid contents**

| No. | phospholipid lipid content % | Particle size (nm) | PDI | Encapsulation efficiency (%) | Fluorescence intensity |
|---|---|---|---|---|---|
| B1 | 0 | 115.2 | 0.09 | 93.5 | 2.86E+11 |
| B2 | 2.5 | 106.7 | 0.09 | 95.9 | 2.09E+11 |
| B3 | 5 | 97.0 | 0.11 | 96.9 | 2.23E+11 |
| B4 | 7.5 | 115.8 | 0.12 | 95.6 | 7.47E+ 10 |
| B5 | 10 | 101.6 | 0.10 | 95.9 | 4.09E+10 |
| B6 | 12.5 | 98.1 | 0.08 | 95.8 | 2.33E+10 |
| B7 | 15 | 93.9 | 0.06 | 96.0 | 1.34E+10 |
| ALC-0315 | 10 | 71.3 | 0.08 | 94.1 | 1.26E+10 |
| SM-102 | 10 | 91.9 | 0.09 | 96.4 | 9.86E+9 |

Finally, it should be noted that the above examples are only used to illustrate the technical solutions of the present invention, rather than to limit it. Although the present invention has been described in detail with reference to the aforementioned examples, those skilled in the art should understand that they can still modify the technical solutions described in the aforementioned examples, or make equivalent replacements for some of the technical features therein. However, these modifications or replacements do not deviate the essence of the corresponding technical solutions from the spirit and scope of the technical solutions of the examples of the present invention.

## Claims

1. An amino lipid having the structure as shown in formula (I), or an isomer, a pharmaceutically acceptable salt, a prodrug or a solvate thereof: 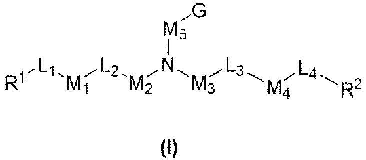
wherein G is selected from H, OR, CN, -C(=O)OR', -OC(=O)R', -C(=O)NR'R", -NR'C(=O)R", -NR'R", or a cycloalkyl comprising at least one heteroatom, wherein the substitutable carbon atoms or heteroatoms in the cycloalkyl are unsubstituted or substituted by one or more groups of hydroxyl, C₁-C₄ alkyl, C₂-C₄ alkenyl, C₃-C₈ cycloalkyl, or C₃-C₈ cycloalkenyl;
M₁, M₂, M₃, and M₄ are identical or different from each other, and each independently selected from C₁-C₂₄ alkylene, C₃-C₂₄ cycloalkylene, C₂-C₂₄ alkenylene, or C₃-C₂₄ cycloalkenylene;
R¹ and R² are identical or different from each other, and each independently selected from H, C₁-C₂₄ alkyl, C₃-C₂₄ cycloalkyl, C₂-C₂₄ alkenyl, or C₃-C₂₄ cycloalkenyl;
L₁, L₂, L₃, and L₄ are identical or different from each other, and each independently selected from -C(=O)O-, -OC(=O)-, -C(=O)S-, -SC(=O)-, -C(=O)NR-, -NRC(=O)-, -S(=O)-, -OS(=O)₂-, -S(=O)₂O-, -O-, -S-, or -S-S-;
R, R', and R" are identical or different from each other, and each independently selected from H, C₁-C₁₀ alkyl, C₃-C₁₀ cycloalkyl, C₃-C₁₀ alkenyl, C₃-C₁₀ cycloalkenyl, C₁-C₁₀ alkyl terminated with a tertiary amino group, C₃-C_{lO} cycloalkyl terminated with a tertiary amino group, C₃-C₁₀ alkenyl terminated with a tertiary amino group, or cycloalkyl comprising at least one heteroatom, the cycloalkyl being unsubstituted or substituted with one or more groups of C₁-C₄ alkyl, C₂-C₄ alkenyl, C₃-C₈ cycloalkyl, or C₃-C₈ cycloalkenyl;
M₅ groups are each independently selected from a single bond, C₁-C₁₆ alkylene, C₂-C₁₆ alkenylene, C₃-C₈ cycloalkylene, or C₃-C₈ cycloalkenylene.

2. The amino lipid according to claim 1, wherein G is selected from H, OR, CN, -C(=O)OR', -OC(=O)R', -C(=O)NR' R", -NR'C(=O)R", -NR'R" or cycloalkyl comprising at least one heteroatom, wherein the substitutable carbon atoms or heteroatoms in the cycloalkyl are unsubstituted or substituted by one or more groups of C₁-C₄ alkyl, C₂-C₄ alkenyl, C₃-C₈ cycloalkyl or C₃-C₈ cycloalkenyl; R, R', and R" are identical or different from each other, and each independently selected from H, C₁-C₁₀ alkyl, C₃-C_{lO} cycloalkyl, C₃-C₁₀ alkenyl or C₃-C_{lO} cycloalkenyl, or cycloalkyl comprising at least one heteroatom; the cycloalkyl is unsubstituted or substituted by one or more groups of C₁-C₄ alkyl, C₂-C₄ alkenyl, C₃-C₈ cycloalkyl, or C₃-C₈ cycloalkenyl.

3. The amino lipid according to claim 1 or 2, wherein in the formula (I), G is selected from H, OR, CN, -C(=O)OR', -OC(=O)R', -C(=O)NR' R", -NR'C(=O)R", -NR'R", or cycloalkyl comprising at least one heteroatom; wherein the heteroatom is O or N; and the cycloalkyl is unsubstituted or substituted with one or more groups of C₁-C₄ alkyl, C₃-C₈ cycloalkyl or hydroxyl; R, R', and R" are identical or different from each other, and each independently selected from H, C₁-C₈ alkyl, C₃-C₈ cycloalkyl, C₃-C₈ alkenyl, or C₃-C₈ cycloalkenyl, C₁-C₁₀ alkyl terminated with a tertiary amino group, C₃-C_{lO} cycloalkyl terminated with a tertiary amino group, or a cycloalkyl comprising at least one heteroatom, the cycloalkyl being unsubstituted or substituted with one or more groups of C₁-C₄ alkyl and C₃-C₈ cycloalkyl.

4. The amino lipid according to claim 3, wherein in the formula (I), G is selected from H, OR or NR'R", wherein R' and R" are identical or different from each other, and each independently selected from H or C₁-C₄ alkyl; or
G is selected from substituted or unsubstituted 5-membered oxacycloalkyl, substituted or unsubstituted 5-membered azacycloalkyl, substituted or unsubstituted 6-membered azacycloalkyl, substituted or unsubstituted 6-membered diazocycloalkyl, or substituted or unsubstituted 6-membered azaoxacycloalkyl.

5. The amino lipid according to claim 4, wherein in the formula (I), when G is selected from substituted 6-membered diazocycloalkyl, the substituent is positioned on the nitrogen atom that is not linked with M₅.

6. The amino lipid according to claim 3, wherein in the formula (I), M₅ is selected from a single bond, C₂-C₁₆ alkylene, C₂-C₁₆ alkenylene, C₄-C₈ cycloalkylene, or C₃-C₈ cycloalkenylene.

7. The amino lipid according to claim 6, wherein in formula (I), M₅ is selected from a single bond, C₂-C₁₆ alkylene, or C₄-C₆ cycloalkylene.

8. The amino lipid according to claim 7, wherein in the formula (I), the 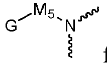 formed by linking of M₅ and G is one selected from A1 to A38:

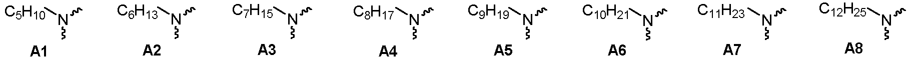

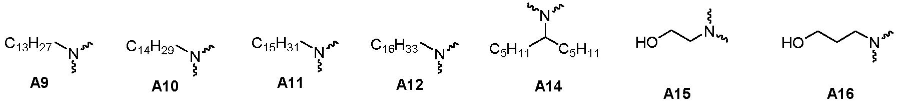

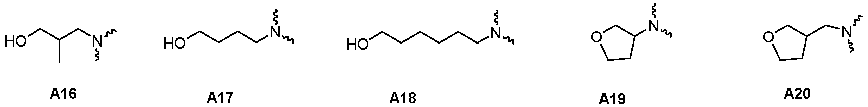

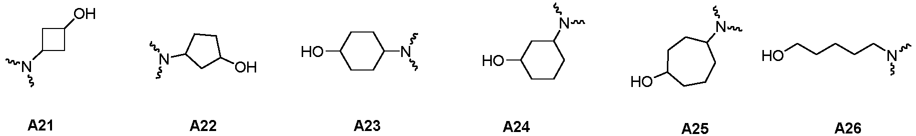

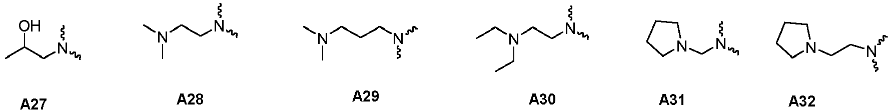

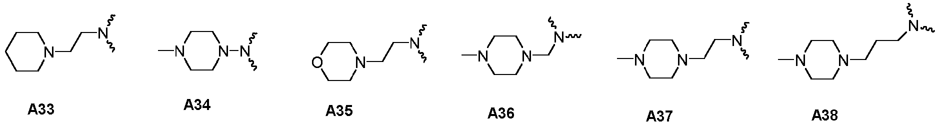

9. The amino lipid according to claim 7, wherein in the formula (I), the 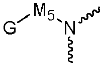 formed by linking of M₅ and G is one selected from A39 to A52:

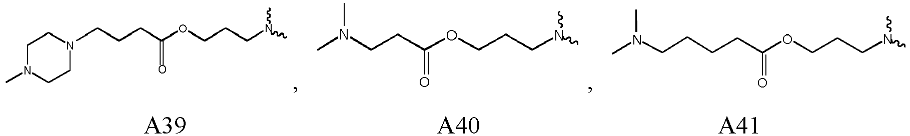

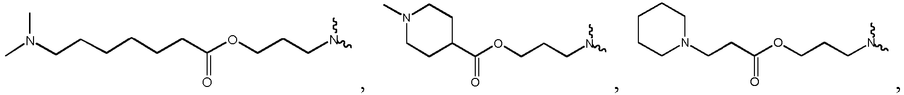

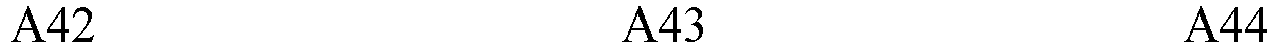

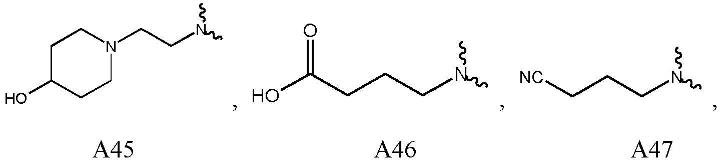

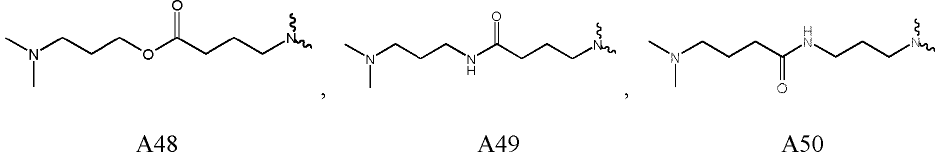

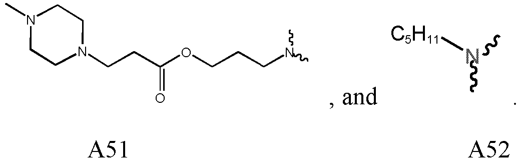

10. The amino lipid according to claim 8, wherein the 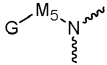 is one selected from A1-A18, A22-A24, and A28-A38.

11. The amino lipid according to claim 10, wherein the 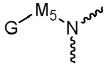 is one selected from A15, A16, A17, A23, A29, A30, A33, A37, A38, and A42.

12. The amino lipid according to any one of claims 1 to 11, wherein in the formula (I), L₁, L₂, L₃, and L₄ are identical or different, and independently selected from -C(=O)O-, -OC(=O)-, -C(=O)NR-, -NRC(=O)-, or -S-S-;
preferably, L₁ and L₄ are the same and are -C(=O)O- or -OC(=O)-.

13. The amino lipid according to claim 12, wherein when L₁, L₂, L₃, and L₄ are independently selected from -C(=O)NR- or -NRC(=O)-, R is independently selected from H or C₁-C₁₀ alkyl.

14. The amino lipid according to any one of claims 1 to 13, wherein in the formula (I), M₁, M₂, M₃, and M₄ are identical or different, M₁ and M₄ are each independently selected from branched-chain C₄-C₂₂ alkylene, branched-chain C₄-C₂₂ cycloalkylene, branched-chain C₄-C₂₂ alkenylene, or branched-chain C₄-C₂₂ cycloalkenylene, and M₂ and M₃ are each independently selected from C₄-C₂₂ alkylene, C₄-C₂₂ cycloalkylene, C₄-C₂₂ alkenylene, or C₄-C₂₂ cycloalkenylene.

15. The amino lipid according to claim 14, wherein in the formula (I), M₂ is the same as M₃ and is a C₄-C₂₂ alkylene; and/or
M₁ is the same as M₄, and is a branched-chain C₄-C₂₂ alkylene.

16. The amino lipid according to any one of claims 2 to 15, wherein in the formula (I), R¹ and R² are identical or different, and each independently selected from C₄-C₂₂ alkyl or C₄-C₂₂ alkenyl.

17. The amino lipid according to claim 16, wherein in the formula (I), the R¹-L₁-M₁-L₂-M₂- fragment is R¹-C(=O)O-M₁-OC(=O)-M₂-, the R²-L₄-M₄-L₃-M₃- fragment is R²-C(=O)O-M₄-OC(=O)-M₃-.

18. The amino lipid according to claim 17, wherein the structure as shown in formula (I) is one selected from the following structures:

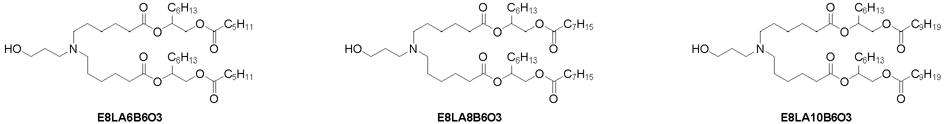

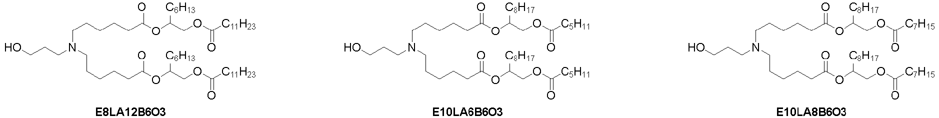

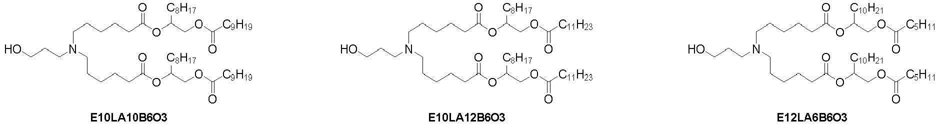

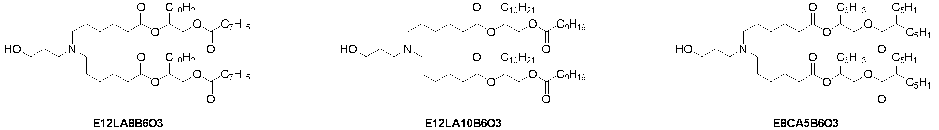

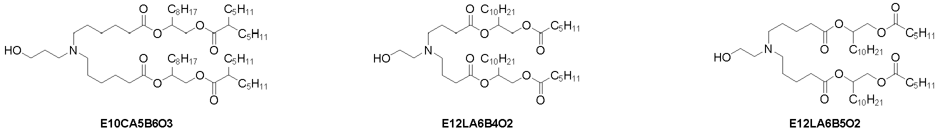

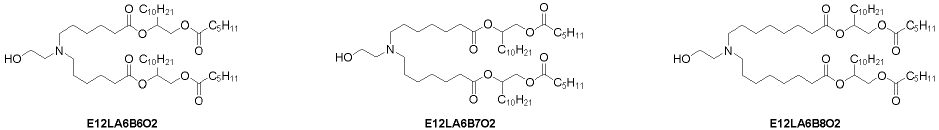

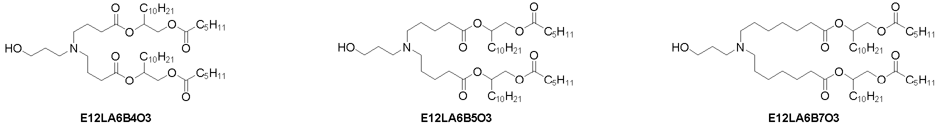

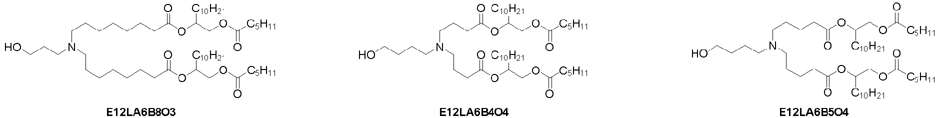

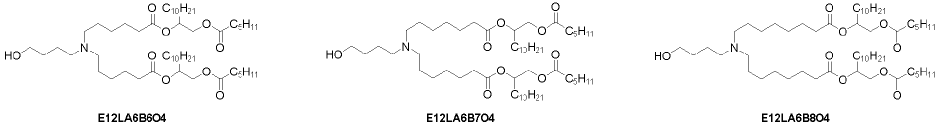

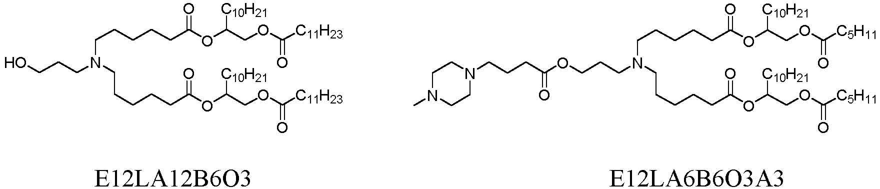

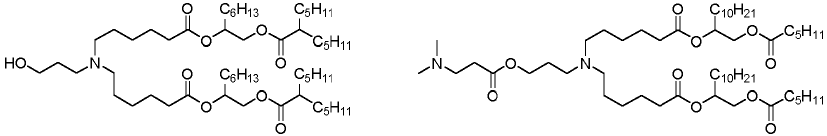

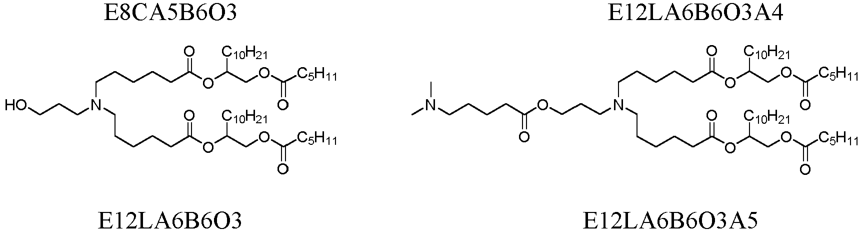

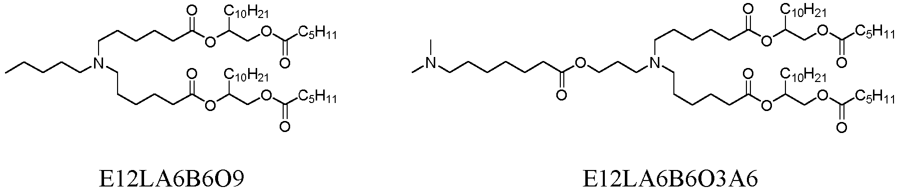

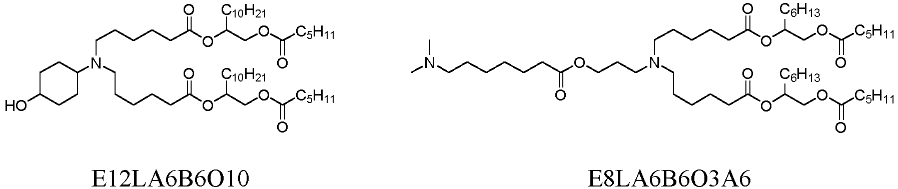

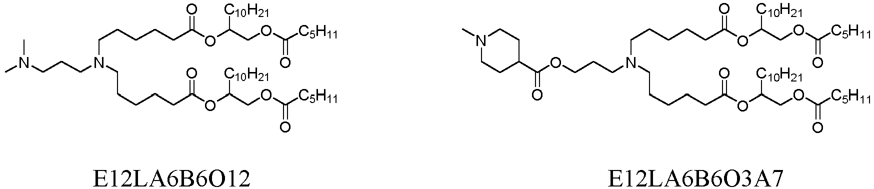

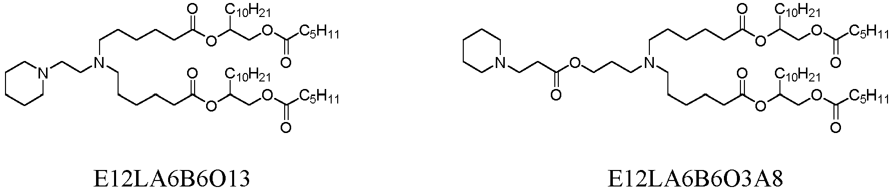

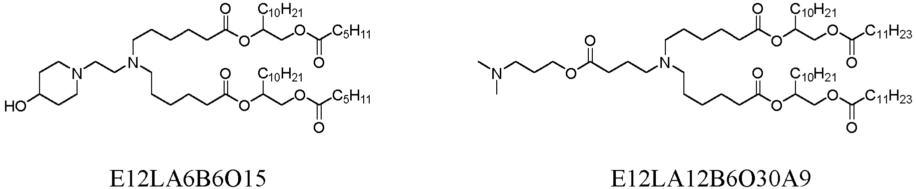

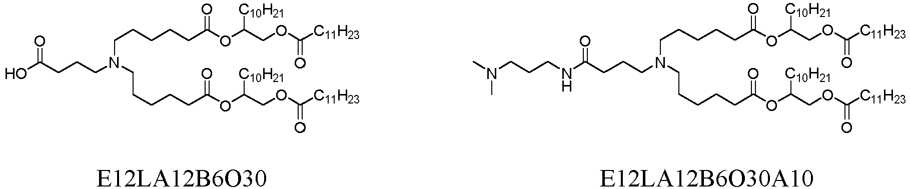

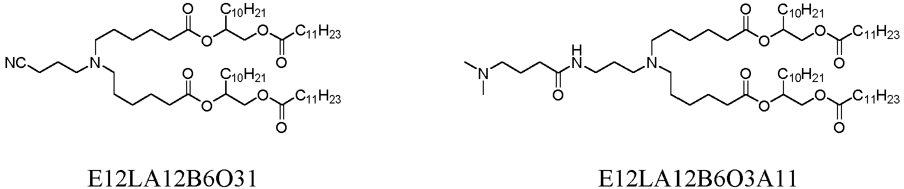

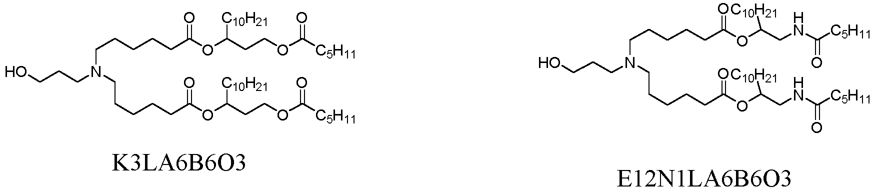

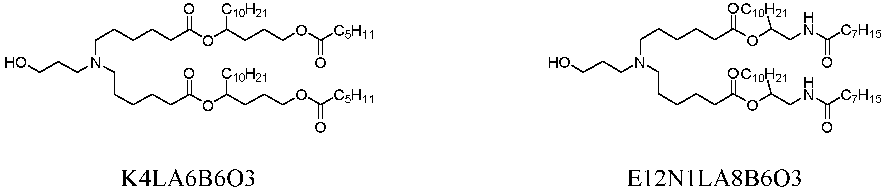

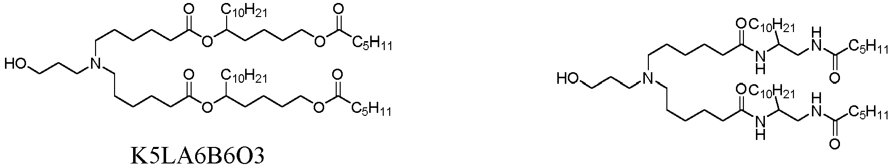

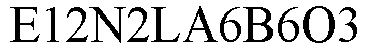

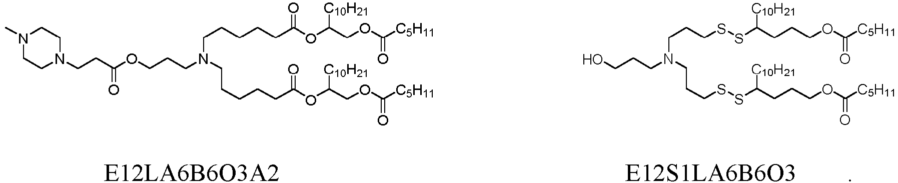

19. A method for preparing the amino lipid according to any one of claims 1 to 18, comprising the following steps:
S1: subjecting an epoxide compound and a carboxylic acid to ring-opening reaction to prepare R¹-L₁-M₁-OH as intermediate 1;
S2: subjecting the intermediate 1 and a carboxylic acid compound as a starting material to a condensation reaction in the presence of a condensation agent to give R¹-L₁-M₁-L₂-M₂-leaving group as intermediate 2; and
S3: subjecting the intermediate 2 and an amino compound as a starting material to one or more substitution reactions, to obtain the target product; or
S1': performing oxidation reaction of a diol undergoing TBS protection, and subjecting the oxidation product and a Grignard reagent to addition reaction, to give HO-M₁-OTBS as intermediate 1';
S2': subjecting the intermediate 1' and a carboxylic acid compound as a starting material to a condensation reaction in the presence of a condensation agent, to give TBSO-M₁-L₂-M₂-leaving group as intermediate 2';
S3': removing the protective group of TBS from the intermediate 2' TBSO-M₁-L₂-M₂-leaving group, allowing the resultant to react with a carboxylic acid to generate R¹-L₁-M₁-L₂-M₂-leaving group as intermediate 2; and
S4': subjecting the intermediate 2 and an amino compound as a starting material to one or more substitution reactions, to obtain the target product.

20. A lipid nanoparticle, comprising the amino lipid according to any one of claims 1 to 18.

21. The lipid nanoparticle according to claim 20, wherein the lipid nanoparticle further comprises a steroid, a neutral lipid and/or a polymer-conjugated lipid;
wherein the polymer-conjugated lipid has a chemical formula of P-Y-L, in which P is a hydrophilic polymer moiety, Y is an optional linker, and L is a lipid moiety.

22. The lipid nanoparticle according to claim 21, wherein the steroid is cholesterol; and/or
the neutral lipid is a phospholipid; and/or
the polymer-conjugated lipid is PEGylated lipids.

23. The lipid nanoparticle according to claim 21, wherein a molar ratio of the amino lipid, the steroid, the neutral lipid, and the polymer-conjugated lipid in the lipid nanoparticles is 30-70:30-65:0-30:0.2-5.

24. A pharmaceutical composition, comprising the lipid nanoparticle according to any one of claims 20 to 23 and a pharmaceutically acceptable carrier.

25. A method for treating or preventing infectious diseases, cancer, genetic diseases, allergy, toxicity, and autoimmune diseases using the amino lipid according to any one of claims 1 to 18, the lipid nanoparticle according to any one of claims 20 to 23, or the pharmaceutical composition according to claim 24.

26. The method according to claim 25, wherein the cancer includes lung cancer, gastric cancer, liver cancer, esophageal cancer, colon cancer, pancreatic cancer, brain cancer, lymphoma, leukaemia, and prostate cancer.

27. A method for gene therapy, genetic vaccination, antisense therapy, delivery of nucleic acids, or treatment through RNA interference, by using the amino lipid according to any one of claims 1 to 18, the lipid nanoparticle according to any one of claims 20 to 23, or the pharmaceutical composition according to claim 24.

28. Use of the amino lipid according to any one of claims 1 to 18, the lipid nanoparticle according to any one of claims 20 to 23, or the pharmaceutical composition according to claim 24 in the preparation of a medicament for treating or preventing infectious diseases, cancer, genetic diseases, allergy, toxicity, and autoimmune diseases.

29. The use according to claim 28, wherein the cancer includes lung cancer, gastric cancer, liver cancer, esophageal cancer, colon cancer, pancreatic cancer, brain cancer, lymphoma, leukaemia, and prostate cancer.

30. Use of the amino lipid according to any one of claims 1 to 18, the lipid nanoparticle according to any one of claims 20 to 23, or the pharmaceutical composition according to claim 24 in the preparation of a medicament for gene therapy, genetic vaccination, antisense therapy, delivery of nucleic acids, or treatment through RNA interference.

31. A method for delivering a medicament to a subject, comprising administering to the subject a medicament formulated in the lipid nanoparticle according to any one of claims 20 to 23.

32. Use of the amino lipid according to any one of claims 1 to 18 for treating or preventing infectious diseases, cancer, genetic diseases, allergy, toxicity, and autoimmune diseases.
